# EUROPEAN PATENT APPLICATION

(11) **EP 4 282 879 A2**
(43) Date of publication of application: **29.11.2023**
(21) Application number: 23201805.1
(22) Date of filing: 30.11.2012
(51) Int. Cl.: C07K 16/00

(54) **USE OF FERTILE TRANSGENIC ANIMALS FOR PRODUCING ANTIBODIES BEARING HUMAN VARIABLE REGIONS**

(30) Priority: 02.12.2011 US 201113310431; 21.12.2011 GB 201122047; 09.03.2012 US 201213416684
(62) Divisional of application: 17196214.5
(71) Applicant: Kymab Ltd., Cambridge CB22 3AT (GB)
(72) Inventor: FRIEDRICH, Glenn A., Cambridge, CB22 3AT (GB); LEE, E-Chiang, Cambridge, CB22 3AT (GB)
(74) Representative: CMS Cameron McKenna Nabarro Olswang LLP

(57) **Abstract**

The present invention relates *inter alia* to fertile non-human vertebrates such as mice and rats useful for producing antibodies bearing human variable regions, in which endogenous antibody chain expression has been inactivated. This inactivation leads to loss of ADAM6 genes located within the rodent heavy chain locus, said genes being crucial for fertility. Introduction of functional copies of said ADAM6 genes allows production of fertile mice.

## Description

The present invention relates *inter alia* to fertile non-human vertebrates such as mice and rats useful for producing antibodies bearing human variable regions, in which endogenous antibody chain expression has been inactivated.

### BACKGROUND

Antibody-generating non-human vertebrates such as mice and rats that comprise one or more transgenic antibody loci encoding variable regions are generally known in the art, and by way of example reference is made to WO2011004192, US7501552, US6673986, US6130364, WO2009/076464 and US6586251, the disclosures of which are incorporated herein by reference in their entirety.

Using embryonic stem cell (ES cell) technology, the art has provided non-human vertebrates, such as mice and rats, bearing transgenic antibody loci from which human or chimaeric antibodies can be generated *in vivo* following challenge with human antigen. Such antibodies usefully bear human variable regions in their heavy chains and optionally also in their light chains. In order to avoid complications of endogenous antibody heavy chain expression at the same time, the genomes of such vertebrates are typically engineered so that endogenous heavy chain expression is inactivated. Techniques for doing this involve the deletion of all or part of the endogenous heavy chain VDJ region simultanesously with the insertion of human VDJ gene segments or in a separate step (eg, see WO2009076464 and WO2002066630). Such deletion entails the deletion of VH and D gene segments along with the intervening sequences. In doing so, the endogenous ADAM6 genes are deleted.

The ADAM6 gene encodes a protein belonging to the A disintegrin and metalloprotease (ADAM) family. ADAM family members are transmembrane glycoproteins that contain conserved multi-domains such as pro-domain, metalloprotease, disintegrin, cysteine-rich, epidermal growth factor (EGF)-like, transmembrane, and cytoplasmic tail domains. The ADAM family has been shown to be involved in cell adhesion [1-5 ] in various biological progress.

In mouse, there are two copies of ADAM6 (ADAM6a, ADAM6b) located between the VH and D gene segments in the IgH locus of chromosome 12 (in the intervening region between mouse V_{H}5-1 and D1-1 gene segments). These two adjacent intronless ADAM6 genes have 95% nucleotide sequence identity and 90% amino acid identity. In human and rat, there is only one ADAM6 gene. Expression pattern analysis of mouse ADAM6 shows that it is exclusively expressed in testis [6]. Although ADAM6 transcripts can be detected in lymphocytes, it is restricted to the nucleus, suggesting that the transcription of ADAM6 gene in particular is due to transcriptional read-through from the D region rather than active messenger RNA production [7].

Mature ADAM6 protein is located on the acrosome and the posterior regions of sperm head. Notably, ADAM6 forms a complex with ADAM2 and ADAM3, which is required for fertilization in mice [8]. Reference [9] implicates ADAM6 in a model where this protein interacts with ADAM3 after ADAM6 is sulphated by TPST2, sulphation of ADAM6 being critical for stability and/or complex formation involving ADAM6 and ADAM3, and thus ADAM6 and ADAM3 are lost from *Tpst2*-null sperm. The study observes that *Tpst2*-deficient mice have male infertility, sperm mobility defects and possible abnormalities in sperm-egg membrane interactions.

Thus, the maintenance of ADAM6 expression in sperm is crucial for fertility. Thus, it is thought that transgenic male mice and rats in which ADAM6 genes have been deleted are not viably fertile. This hampers breeding of colonies and hampers the utility of such mice as transgenic antibody-generating platforms. It would be desirable to provide improved non-human transgenic antibody-generating vertebrates that are fertile.

### References

[1] Primakoff P, Myles DG. The ADAM gene family: surface proteins with adhesion and protease activity. Trends Genet. 2000 Feb;16(2):83-7.
[2] Evans JP. Fertilin beta and other ADAMs as integrin ligands: insights into cell adhesion and fertilization. Bioessays. 2001 Jul;23(7):628-39.
[3] Primakoff P, Myles DG. Penetration, adhesion, and fusion in mammalian sperm-egg interaction. Science. 2002 Jun 21;296(5576):2183-5.
[4] Talbot P, Shur BD, Myles DG. Cell adhesion and fertilization: steps in oocyte transport, sperm-zona pellucida interactions, and sperm-egg fusion. Biol Reprod. 2003 Jan;68(1):1-9.
[5] Huovila AP et. al., Shedding light on ADAM metalloproteinases. Trends Biochem Sci. 2005 Jul;30(7):413-22.
[6]. Choi I,et. al., Characterization and comparative genomic analysis of intronless Adams with testicular gene expression. Genomics. 2004 Apr;83(4):636-46.
[7]. Featherstone K, Wood AL, Bowen AJ, Corcoran AE. The mouse immunoglobulin heavy chain V-D intergenic sequence contains insulators that may regulate ordered V(D)J recombination. J Biol Chem. 2010 Mar 26;285(13):9327-38. Epub 2010 Jan 25.
[8]. Han C, et. al., Comprehensive analysis of reproductive ADAMs: relationship of ADAM4 and ADAM6 with an ADAM complex required for fertilization in mice. Biol Reprod. 2009 May;80(5):1001-8. Epub 2009 Jan 7.
[9]. Marcello et al, Lack of tyrosylprotein sulfotransferase-2 activity results in altered sperm-egg interactions and loss of ADAM3 and ADAM6 in epididymal sperm, J Biol Chem. 2011 Apr 15;286(15):13060-70. Epub 2011 Feb 21.

### SUMMARY OF THE INVENTION

To this end, the present invention provides:-
A method of making a fertile non-human vertebrate (eg, a mouse) that is homozygous for a transgenic antibody heavy chain locus,
the mouse having a genome that
   (a) comprises each transgenic heavy chain locus on a respective copy of chromosome 12 (or equivalent chromosome for said vertebrate); and
   (b) is inactivated for endogenous antibody heavy chain expression;
the method comprising the steps of
   (c) constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 (or equivalent chromosome for said vertebrate) so that the human gene segments are operably connected upstream of a mouse or human endogenous heavy chain constant region (optionally Cmu and/or Cgamma);
   (d) simultaneously or separately from step (c), deleting all or part of the mouse endogenous heavy chain VDJ region of said chromosome 12 to inactivate endogenous antibody heavy chain expression, wherein the deletion includes mouse ADAM6-encoding nucleotide sequence;
   (e) simultaneously or separately from step (c) or (d), inserting into the ES cell genome one or more ADAM6-encoding nucleotide sequences; and
   (f) developing the ES cell into a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

In a second configuration, the invention provides a method of making a fertile non-human vertebrate (eg, a mouse) that is homozygous for a transgenic antibody heavy chain locus,
the mouse having a genome that
   (a) comprises each transgenic heavy chain locus on a respective copy of chromosome 12 (or equivalent chromosome for said vertebrate); and
   (b) is inactivated for endogenous antibody heavy chain expression;
the method comprising the steps of
   (c) constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse or human endogenous heavy chain constant region (optionally Cmu and/or Cgamma);
   (d) simultaneously or separately from step (c), deleting all or part of the mouse endogenous heavy chain VDJ region of said chromosome 12 to inactivate endogenous antibody heavy chain expression, wherein the deletion includes mouse ADAM6-encoding nucleotide sequences;
   (e) developing the ES cell into a child mouse or progeny thereof whose genome comprises a said transgenic heavy chain locus;
   (f) deriving a second ES cell from said mouse and inserting into the genome of said second ES cell one or more ADAM6-encoding nucleotide sequences; and
   (g) developing the second ES cell into a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

In a third configuration, the invention comprises a method of making a fertile non-human vertebrate (eg, a mouse) that is homozygous for a transgenic antibody heavy chain locus,
the mouse having a genome that
   (a) comprises each transgenic heavy chain locus on a respective copy of chromosome 12 (or equivalent chromosome for said vertebrate); and
   (b) is inactivated for endogenous antibody heavy chain expression;
the method comprising the steps of
   (c) constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse or human endogenous heavy chain constant region (optionally Cmu and/or Cgamma);
   (d) simultaneously or separately from step (c), deleting all or part of the mouse endogenous heavy chain VDJ region of said chromosome 12 to inactivate endogenous antibody heavy chain expression, wherein the deletion includes mouse ADAM6-encoding nucleotide sequences;
   (e) developing the ES cell into a child mouse or progeny thereof whose genome comprises a said transgenic heavy chain locus; and
   (f) by breeding using said child mouse (or progeny) and a further mouse whose genome comprises one or more ADAM6-encoding nucleotide sequences, developing a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

In a fourth configuration, the invention provides a fertile non-human vertebrate (optionally a male) that is homozygous for a transgenic antibody heavy chain locus, the vertebrate having a genome that
(i) comprises each transgenic heavy chain locus on a respective copy of a first chromosome; and
(ii) is inactivated for endogenous antibody heavy chain expression;
wherein each first chromosome of the genome comprises
(iii) a transgenic antibody heavy chain locus comprising one or more human VH gene segments, one or more human D gene segments and one or more human JH gene segments operably connected upstream of a mouse or human heavy chain constant region (optionally Cmu and/or Cgamma);
(iv) a deletion of all or part of the endogenous heavy chain VDJ region of said chromosome to inactivate endogenous antibody heavy chain expression, wherein the deletion includes ADAM6; and
wherein the genome comprises
(v) an insertion of one or more expressible ADAM6-encoding nucleotide sequences.

Thus, ADAM6 resides on each said first chromosome in a wild-type fertile non-human vertebrate, but inactivation of endogenous heavy chain expression involves deletion of ADAM6 that is co-located with the deleted heavy chain gene segments on the same chromosome. For example, use of homologous recombination precisely to replace endogenous heavy chain VDJ with human VDJ gene segments as in the prior art deletes endogenous ADAM6, thus affecting fertility. In the mouse, this happens when deletion of all or part of the endogenous heavy chain VDJ region on chromosome 12 is deleted to inactivate endogenous heavy chain expression. In the rat, this happens when deletion of all or part of the endogenous heavy chain VDJ region on chromosome 6 is deleted to inactivate endogenous heavy chain expression. The invention inserts ADAM6 into the vertebrate genome in order to restore fertility.

In one aspect of the fourth configuration, the vertebrate is a mouse and each first chromosome is a chromosome 12.

Thus, in one aspect of the fourth configuration, the vertebrate is a rat and each first chromosome is a chromosome 6.

The invention provides a method of making a fertile non-human vertebrate, eg, mouse or rat, that is homozygous for a transgenic antibody heavy chain locus by carrying out steps (a) to (d) in an ES cell and using ES cell genome technology developing a final non-human vertebrate having a genome comprising an inserted ADAM6-encoding nucleotide sequence (in homozygous or heterozygous state) and said transgenic heavy chain locus in homozygous state, wherein endogenous ADAM6 has been deleted. The invention also provides a fertile non-human vertebrate, eg, mouse or rat, that is made by this method, or a fertile male or female progeny thereof.

### BRIEF DESCRIPTION OF THE FIGURES

Figures 1a & 1b: Schematic for endogenous IgH inactivation and retention of Adam6 by translocation;
Figure 2: Schematic for homologous recombination replacement of endogenous (mouse) IgH loci gene segments with human gene segments and accompanying deletion of Adam6 genes;
Figure 3: Schematic for RMGR replacement of endogenous (mouse) IgH loci gene segments with human gene segments and accompanying deletion of Adam6 genes;
Figure 4: Schematic for the creation and targeting of a deletion vector;
Figure 5: Schematic for the creation of a targeting vector containing Adam6 genes;
Figure 6: Schematic for the creation of IgH BAC containing Adam6 genes.

### DETAILED DESCRIPTION OF THE INVENTION

The invention provides a method of making a fertile non-human vertebrate (eg, a mouse) that is homozygous for a transgenic antibody heavy chain locus. The final mouse resulting from the method is in one embodiment a male, so that the invention improves upon the prior art male transgenic mice that are infertile as a result of genomic manipulation. Fertile mice produce sperm that can fertilise eggs from a female mouse. Fertility is readily determined, for example, by successfully breeding to produce an embryo or child mouse. In another embodiment, the method of the invention makes a final female mouse. Such females are, of course, useful for breeding to create male progeny carrying ADAM6 and which are fertile.

In the method of this aspect of the invention, the final mouse has a genome that comprises each transgenic heavy chain locus on a respective copy of chromosome 12. The heavy chain loci in wild-type mice are found on chromosomes 12 and, as per the explanation below, the invention entails building a transgenic locus on the same chromosome. In one example, the transgenic locus is a chimaeric locus that comprises human VDJ gene segments inserted upstream of the endogenous mouse constant region (at least the mouse Cmu and/or Cgamma). The human gene segments are operably connected with the constant regions in the present invention so that, after differentiation into a B-cell progeny in a mouse, the B-cell is able to express chimaeric antibodies comprising heavy chains having human variable regions and mouse constant regions. In an alternative aspect of any configuration of the invention, instead of a mouse (or non-human) constant region, each transgenic heavy chain locus comprises said human VDJ gene segments operably connected upstream of a human heavy chain constant region, eg, human Cmu (optionally with a mouse or human Smu with human Cmu) and/or human gamma.

To this end, the method comprises the step of: constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse endogenous heavy chain constant region (optionally Cmu and/or Cgamma). Optionally, the human gene segments are inserted upstream of the endogenous mouse Smu switch and Cmu. This is useful to harness the mouse endogenous regulatory control for class switching from IgM to another type (eg, IgG) antibodies *in vivo* following immunisation of a final mouse with an antigen of interest. In an example, the resultant ES cell is heterozygous for the transgenic heavy chain locus, ie, the transgenic locus is present on one chromosome 12 in the cell. The other chromosome 12 can, for example, bear the endogenous heavy chain locus and optionally this is inactivated (eg, by insertion of a functional marker (eg, neo or hprt) or by deletion of all or part of the locus, such as all or part of the endogenous VDJ region). The heterozygous ES cell can be developed in due course into a mouse that is heterozygous for the heavy chain transgenic locus and using breeding and crossing with other mice also containing a copy of the transgenic heavy chain locus, a resultant progeny can be obtained that is homozygous for the transgenic heavy chain transgene. One or more ADAM6-encoding nucleotide sequences can have been inserted (as described further below) into the genome of one or both of the heterozygous ancestor mice (eg, by insertion of ADAM6 into a respective ES cell that is an ancestor of the ancestor mouse; or by breeding of mice, one of which bears ADAM6, so that the resultant progeny is one of said ancestor mice bearing ADAM6). Alternatively, a progeny mouse that is homozygous for the heavy chain transgene but null for ADAM6 can be crossed with a mouse whose genome contains an ADAM6 gene, and using breeding a progeny that is homozygous for the heavy chain transgene and also contains an ADAM6 gene (in heterozygous or homozygous state) can be obtained. Instead of using just breeding, ES cell genome manipulation can be used to insert an ADAM6-encoding nucleotide sequence into an ES cell derived from a progeny mouse that is homozygous for the heavy chain transgene and a mouse subsequently is developed from the ES cell (or a progeny thereof) so that the final mouse genome is homozygous for the heavy chain transgene and also comprises an ADAM6 gene. Techniques of animal husbandry, crossing, breeding, as well as ES cell (eg, IPS cell) genome manipulation are readily available in the state of the art and will be familiar to the skilled person.

In the method of the present invention, simultaneously or separately from inserting the human gene segments into the ES cell genome, all or part of the mouse endogenous heavy chain VDJ region of said chromosome 12 is deleted to inactivate endogenous antibody heavy chain expression, ie, in a final progeny mouse derived from the ES cell, endogenous antibody heavy chain expression is inactivated. In one embodiment, the endogenous VDJ deletion is carried out simultaneously with the insertion of the human VDJ. For example, one can use homologous recombination in a technique precisely to replace the entire mouse VDJ region (or part thereof including ADAM6-encoding nucleotide sequences) with the human VDJ gene segments. One method (eg, see WO2002066630) is to use a plurality of homologous recombination vectors (eg, bacterial artificial chromosomes; BACs) each bearing one or more human VH and/or D and/or JH segments, in which a vector has homology arms flanking one or more human VH gene segments to be placed at the 5' end of the transgenic heavy chain locus. In this vector, the 5' homology arm can be a sequence corresponding to a mouse genomic sequence immediately 5' of the endogenous heavy chain locus. Using standard homologous recombination, this inserts the human gene segments precisely to replace endogenous mouse gene segments at the 5' position of the endogenous heavy chain locus. Another vector comprises homology arms flanking one or more human JH gene segments (and optionally all or part of the mouse J-C intron) to be placed at the 3' end of the transgenic heavy chain VDJ. In this vector, the 3' homology arm can be a sequence corresponding to a mouse genomic sequence immediately 5' of the endogenous heavy chain Cmu (or another downstream endogenous constant region); alternatively, the 3' homology arm can be a sequence corresponding to all or part of the endogenous J-C intron. Using standard homologous recombination, this inserts the human gene segments from this vector precisely to replace endogenous mouse gene segments at the 3' position of the endogenous heavy chain locus. In one embodiment, the plurality of BACs have overlapping homology arms and can be used to replace the endogenous VDJ with human VDJ gene segments, eg, see WO2009076464). In another embodiment, one or more of these homologous recombination techniques can be generally used, with the modification that the human VDJ is inserted immediately downstream (3') of the endogenous VDJ region (eg, inserted in the endogenous J-Cmu intron) and in one or more subsequent steps the endogenous VDJ (or part thereof comprising the ADAM6-encoding nucleotide sequences) is deleted, eg, using standard site-specific recombination (eg, cre/lox), transposon (eg, piggyBac transposon) or homologous recombination techniques. In another embodiment, the human VDJ is inserted 5' (eg, immediately 5' or within 100kb 5') of the first mouse VH gene segment and in one or more subsequent steps the endogenous VDJ (or part thereof comprising the ADAM6-encoding nucleotide sequences) is deleted.

In one embodiment of any configuration, aspect or embodiment of the present invention (eg, method and vertebrates of the invention), the endogenous VDJ (or part thereof including ADAM6-encoding nucleotide sequence(s)) is deleted from the chromosome by translocation to a different chromosome species. For example, the different chromosome is chromosome 15. Translocation between chromosomes 12 and 15 in a mouse, for example, is desirable since it is known from published observations that translocation between the heavy chain locus on chromosome 12 and c-*myc* on chromosome 15 is possible (see, eg, Science 24 December 1982: Vol. 218 no. 4579 pp. 1319-1321; "Mouse c-myc oncogene is located on chromosome 15 and translocated to chromosome 12 in plasmacytomas"; Crews *et al*)*.* Thus, in one example where the vertebrate is a mouse, the endogenous VDJ (or part thereof) is deleted from chromosome 12 by translocation to a chromosome 15. In another example, where the vertebrate is a rat, the endogenous VDJ (or part thereof) is deleted from chromosome 6 by translocation to a chromosome 15. Thus, in the final fertile mouse or mouse progeny of the invention, endogenous heavy chain expression is inactivated by translocation of at least part of the endogenous heavy chain loci VDJ to a non-wild-type chromosome (ie, not a chromosome 12). Thus, in the final fertile rat or rat progeny of the invention, endogenous heavy chain expression is inactivated by translocation of at least part of the endogenous heavy chain loci VDJ to a non-wild-type chromosome (ie, not a chromosome 6). In this case, the translocated endogenous VDJ (or part) is retained in the animal's genome, but is rendered non-functional for endogenous heavy chain expression. This is advantageous because the endogenous ADAM6 genes are deleted from the wild-type chromosomal location to effect inactivation, but are then inserted into the genome elsewhere on an entirely different chromosomal species (ie, one not harbouring an antibody heavy chain locus) by translocation in a way that enables the inserted endogenous ADAM6 genes to function (and thus give fertility in downstream animals) without re-activating endogenous heavy chain expression. Thus, translocation enables inactivation with concomitant retention of endogenous, wild-type ADAM6 genes to provide for fertility in resultant animals. This perfectly tailors the ADAM6 genes to the animal's genome (since it is the endogenous sequence), and also in one embodiment enables transfer of each inserted endogenous ADAM6 genes together with its endogenous promoter (and any other control elements such as enhancers). Thus, in one embodiment inactivation is carried out by the deletion of a chromosomal sequence (eg, sequence of chromosome 12 in a mouse or 6 in a rat) comprising one or more ADAM6 genes including respective promoter(s) and this is inserted by translocation to a chromosome that does not comprise a heavy chain locus (eg, in a mouse a chromosome other than a chromosome 12; in a rat a chromosome other than a chromosome 6). This can be achieved, for example by translocating at least the DNA immediately flanked by the 3' most endogenous VH gene segment and the 5' most endogenous D segment. In one example, where the non-human vertebrate is a mouse, the translocated DNA comprises or constists of DNA from mouse V_{H}5-1 to D1-1 gene segments. In an embodiment, the entire endogenous VD region is translocated; in another embodiment the entire VDJ region is translocated, in either case this will also translocate the embedded endogenous ADAM6 genes.

All of the techniques described herein with reference to a mouse also apply to other non-human vertebrates where ADAM6 will be deleted along with endogenous VDJ, eg, where the ADAM6 is embedded in the endogenous VDJ region. For example, the techniques can be applied to another transgenic murine species. The techniques can be applied to a transgenic rat. The disclosure, throughout, is to be read with this in mind, so that discussion relating to transgenic mice is equally applicable to making other non-human transgenic animals. Thus, for example, where a mouse chromosome 12 is mentioned and the making of a transgenic mouse, the disclosure herein can be read in the alternative to the making of a transgenic rat, and in this case rat chromosome 6 is intended.

In all cases, the deletion in the endogenous VDJ region on a chromosome preferably includes a deletion of all ADAM6-encoding nucleotide sequences. Thus, when the vertebrate is a mouse, ADAM6a and ADAM6b are deleted. For example, the DNA immediately flanked by the 3' most endogenous VH gene segment and the 5' most endogenous D segment is deleted. In one example, where the non-human vertebrate is a mouse, the DNA from mouse V_{H}5-1 to D1-1 gene segments is deleted.

The invention provides a method of making a fertile non-human vertebrate, eg, mouse or rat, that is homozygous for a transgenic antibody heavy chain locus by carrying out steps (a) to (d) in an ES cell and using ES cell genome technology developing a final non-human vertebrate having a genome comprising an inserted ADAM6-encoding nucleotide sequence (in homozygous or heterozygous state) and said transgenic heavy chain locus in homozygous state, wherein endogenous ADAM6 has been deleted. The invention also provides a fertile non-human vertebrate, eg, mouse or rat, that is made by this method, or a fertile male or female progeny thereof.

In one aspect, simultaneously or separately from inserting the human VDJ and deleting the endogenous VDJ (or part thereof), the method comprises
- inserting into the ES cell genome one or more ADAM6-encoding nucleotide sequences; and
- developing the ES cell into a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

In another aspect, after inserting the human VDJ and deleting the endogenous VDJ (or part thereof), the method comprises
- developing the ES cell into a child mouse or progeny thereof whose genome comprises one or more of said transgenic heavy chain locus (eg, is homozygous for the transgenic heavy chain locus);
- deriving a second ES cell from said mouse and inserting into the genome of said second ES cell one or more ADAM6-endcoding nucleotide sequences; and
- developing the second ES cell into a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

In another aspect, after inserting the human VDJ and deleting the endogenous VDJ (or part thereof), the method comprises
- developing the ES cell into a child mouse or progeny thereof whose genome comprises a said transgenic heavy chain locus (eg, is homozygous for the transgenic heavy chain locus); and
- by breeding using said child mouse (or progeny) and a further mouse whose genome comprises one or more ADAM6-encoding nucleotide sequences, developing a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

In this aspect, optionally said further mouse is homozygous for ADAM6, eg, the mouse genome comprises ADAM6a and ADAM6b in homozygous state. Optionally said mice are of the same mouse strain.

The skilled person will be aware of techniques for deriving embryonic stem cells. For example, said second ES cell can be generated from an embryo (eg, blastocyst stage) using any standard technique for ES cell generation. For example, reference is made to Proc Natl Acad Sci 1997 May 27; 94(11):5709-12; "The origin and efficient derivation of embryonic stem cells in the mouse"; Brook FA & Gardner RL, the disclosure of which is incorporated herein by reference. The embryo can be said child mouse or a progeny embryo thereof. Other standard ES cell-generating techniques can be used. In another embodiment, the second ES cell is an IPS cell (induced pluripotent stem cell) that is derived from said child mouse or progeny thereof. Reference is made to WO2007069666, WO2008118820, WO2008124133, WO2008151058, WO2009006997 and WO2011027180, which provide guidance on IPS technology and suitable methods, the disclosures of which are incorporated herein in their entirety. The IPS cell can in one example be directly generated (ie, without need for breeding) from a somatic cell of the child mouse or a progeny mouse thereof using standard methods.

The skilled person conversant with ES cell technology will readily know how to develop a child from a transgenic ES cell whose genome has been manipulated. For example, a non-human (eg, mouse) ES cell (such as an ES cell comprising a heavy chain transgenic locus) is implanted into a donor blastocyst (eg, a blastocyst of the same strain of vertebrate as the ES cell). The blastocyst is then implanted into a foster mother where it develops into a child (embryo or a born child). In this way, a plurality of children can be developed, each from a respective modified child ES cell. Siblings can be bred together to achieve crosses providing one or more resultant progeny that are homozygous for the transgenic heavy chain locus.

In one example, a mouse ES cell according to any configuration, aspect or example of the invention an ES cell is developed into a child or progeny by
(f) transferring the ES cell into a donor mouse blastocyst or earlier-stage embryo (eg, pre-morula stage);
(g) implanting the blastocyst or embryo into a foster mouse mother; and
(h) developing the blastocyst or embryo into a child mouse or progeny thereof that is fertile and whose genome is homozygous for said transgenic heavy chain locus and encodes ADAM6.

In any aspect of configuration of the invention, the position of insertion of ADAM6-encoding nucleotide sequence(s) is not limited to the original chromosome (eg, chromosome 12 for a mouse or chromosome 6 for a rat); insertion into another chromosome is possible, or on the original chromosome but spaced away from the wild-type ADAM6 gene location. In an example, an ADAM6 gene is inserted into an original chromosome, eg, when making a transgenic mouse, an ADAM6-encoding nucleotide sequence is inserted into a chromosome 12; when making a transgenic rat, an ADAM6-encoding nucleotide sequence is inserted into a chromosome 6. In one example, an ADAM6-encoding nucleotide sequence is inserted within 20, 15, 10, 5, 4, 3, 2, 1 or 0.5 Mb of one or both transgenic heavy chain loci. This is useful to maximise linkage between the inserted ADAM6 and the transgenic heavy chain locus, to minimise separation of the genes during subsequent meiosis and crossing, eg, during breeding of progeny. Thus, final mice and progeny thereof can retain the fertility advantage of the invention while permitting useful subsequent breeding and crossing to create new animal lines. In another example, an ADAM6-encoding nucleotide sequence is inserted within one or both transgenic heavy chain loci, eg, in the DNA between the 3' most human VH gene segment and the 5' most human D segment, which nature indicates as a permissive permission for harbouring ADAM6.

In any aspect of configuration of the invention, one or more ADAM6 (eg, two) -encoding nucleotide sequences are inserted into the vertebrate genome by ES cell technology and/or by breeding. The inserted ADAM6-encoding nucleotide sequence(s) do not need to be from the same species as the recipient non-human vertebrate. For example, the vertebrate is a mouse and a rat or primate (eg, human) ADAM6-encoding nucleotide sequence is inserted. For example, the vertebrate is a rat and a mouse or primate (eg, human) ADAM6-encoding nucleotide sequence is inserted.

In one embodiment, the vertebrate is a mouse and an ADAM6-encoding nucleotide sequence is inserted on one or both chromosomes 12. For example, mouse ADAM6a and ADAM6b or rat ADAM6 is inserted on one or both chromosomes 12. For example, a mouse ADAM6-encoding nucleotide sequence is inserted between the 3' most human VH gene segment and the 5' most human D segment.

In one embodiment, the vertebrate is a rat and an ADAM6-encoding nucleotide sequence is inserted on one or both chromosomes 6. For example, mouse or rat ADAM6 is inserted on one or both chromosomes 6. For example, a mouse or rat ADAM6-encoding nucleotide sequence is inserted between the 3' most human VH gene segment and the 5' most human D segment.

In any aspect of configuration of the invention, each ADAM6 is expressible. For example, the inserted ADAM6 nucleotide sequence is inserted so that it is operably connected to a promoter (and optionally an enhancer or other regulatory element) for expression. The promoter can be one that is endogenous to the non-human vertebrate, eg, a mouse promoter (eg, one that drives ADAM6 expression in wild-type mice), or it can be exogenous (from a different species). For example, the inserted ADAM6 in the genome is a rat ADAM6 nucleotide sequence operably connected to an endogenous mouse ADAM6 promoter. Alternatively, the inserted ADAM6 in the genome is a mouse ADAM6 nucleotide sequence operably connected to an endogenous rat ADAM6 promoter.

In one embodiment, an ADAM6 nucleotide sequence is inserted which is selected from the group consisting of SEQ ID NO: 1, 2, 3 and 4 (see sequence listing below).

In one embodiment of the method of the invention, the human immunoglobulin gene segments are inserted into the chromosome to replace all or part of the endogenous heavy chain VDJ region, so that insertion of the human gene segments and deletion of the endogenous VDJ DNA from the chromosome or genome take place simultaneously; optionally wherein the entire endogenous VDJ region is replaced. Insertion of the human gene segments is, for example, performed using homologous recombination and/or site-specific recombination (eg, recombinase mediated cassette exchange) to execute the precise replacement. Deletion of the endogenous VDJ (and particularly the entire endogenous VDJ) from the genome is advantageous to totally eliminate the possibility of recombination with constant region gene segments, thus totally eliminating endogenous heavy chain expression with certainty.

In one example of the method of the invention, wherein the vertebrate is a mouse, mouse ADAM6a and ADAM6b-encoding nucleotide sequences are inserted, such that the final fertile mouse can express both ADAM6a and ADAM6b proteins.

In one example of the method of the invention, the genome of the final fertile mouse or progeny is homozygous for each inserted ADAM6-encoding nucleotide sequence. Optionally the genome comprises more than two copies of mouse ADAM6a and/or ADAM6b-encoding nucleotide sequences. Optionally, as an alternative, the genome comprises 2 copies of ADAM6a and one copy (heterozygous) of ADAM6b; or one copy of ADAM6a and 2 copies of ADAM6b.

In another configuration, the invention provides a fertile non-human vertebrate (optionally a male) that is homozygous for a transgenic antibody heavy chain locus, the vertebrate having a genome that
(i) comprises each transgenic heavy chain locus on a respective copy of a first chromosome; and
(ii) is inactivated for endogenous antibody heavy chain expression;
wherein each first chromosome of the genome comprises
(iii) a transgenic antibody heavy chain locus comprising one or more human VH gene segments, one or more human D gene segments and one or more human JH gene segments operably connected upstream of a mouse heavy chain constant region (optionally Cmu and/or Cgamma);
(iv) a deletion of all or part of the endogenous heavy chain VDJ region of said chromosome to inactivate endogenous antibody heavy chain expression, wherein the deletion includes ADAM6; and
wherein the genome comprises
(v) an insertion of one or more expressible ADAM6-encoding nucleotide sequences.

For example, the non-human vertebrate is murine. For example, the non-human vertebrate is a mouse or a rat.

In one aspect, the invention provides a non-human vertebrate such as a mouse (optionally a male mouse) that is homozygous for a transgenic antibody heavy chain locus, the mouse having a genome that
(i) comprises each transgenic heavy chain locus on a respective copy of chromosome 12 (or equivalent chromosome for said vertebrate); and
(ii) is inactivated for endogenous antibody heavy chain expression;
wherein each chromosome 12 of the genome comprises
(iii) a transgenic antibody heavy chain locus comprising one or more human VH gene segments, one or more human D gene segments and one or more human JH gene segments operably connected upstream of a heavy chain constant region (optionally Cmu and/or Cgamma);
(iv) a deletion of all or part of the mouse endogenous heavy chain VDJ region of said chromosome 12 to inactivate endogenous antibody heavy chain expression (later when differentiated into a mouse/B cells), wherein the deletion includes mouse ADAM6-encoding nucleotide sequences (ie, no functional endogenous ADAM6 genes remain in the genome); and
wherein the genome comprises
(v) an insertion of one or more expressible ADAM6-encoding nucleotide sequences.

The considerations of how and where to insert ADAM6 sequences in the animals of the invention are addressed generally above.

The constant region is, eg, a mouse constant region, eg, an endogenous constant region. Thus, when the vertebrate is a mouse, the constant region is an endogenous mouse constant region, eg, a mouse Cmu and/or a mouse Cgamma, optionally with an endogenous mouse or rat Smu switch.

In another aspect, the invention provides a non-human rat (optionally a male rat) that is homozygous for a transgenic antibody heavy chain locus, the rat having a genome that
(i) comprises each transgenic heavy chain locus on a respective copy of chromosome 6; and
(ii) is inactivated for endogenous antibody heavy chain expression;
wherein each chromosome 6 of the genome comprises
(iii) a transgenic antibody heavy chain locus comprising one or more human VH gene segments, one or more human D gene segments and one or more human JH gene segments operably connected upstream of a heavy chain constant region (optionally Cmu and/or Cgamma);
(iv) a deletion of all or part of the rat endogenous heavy chain VDJ region of said chromosome 6 to inactivate endogenous antibody heavy chain expression (later when differentiated into a mouse/B cells), wherein the deletion includes rat ADAM6-encoding nucleotide sequences (ie, no functional endogenous ADAM6 genes remain in the genome); and
wherein the genome comprises
(v) an insertion of one or more expressible ADAM6-encoding nucleotide sequences.

The constant region is, eg, a rat constant region, eg, an endogenous constant region. Thus, when the vertebrate is a rat, the constant region is an endogenous rat constant region, eg, a rat Cmu and/or a rat Cgamma, optionally with an endogenous mouse or rat Smu switch.

In one example of the homozygous mouse or rat of the invention, each inserted ADAM6-encoding nucleotide sequence is on a (i) chromosome 12 wherein the animal is a mouse; or (ii) chromosome 6 wherein the animal is a rat.

In one example of the homozygous mouse or rat of the invention, an inserted ADAM6-encoding nucleotide sequence is inserted (i) within one or both transgenic heavy chain loci or (ii) within 20 Mb of one or both transgenic heavy chain loci.

In one example of the homozygous mouse or rat of the invention, the human gene segments replace all or part of the endogenous VDJ region in each heavy chain locus.

In one example of the homozygous mouse or rat of the invention, the genome comprises inserted expressible mouse ADAM6a and ADAM6b-encoding nucleotide sequences.

In one example of the homozygous mouse or rat of the invention, the genome comprises an inserted expressible rat ADAM6-encoding nucleotide sequence.

In one example of the homozygous mouse or rat of the invention, the genome is homozygous for each inserted ADAM6-encoding nucleotide sequence. Optionally the genome comprises more than two copies of ADAM6-encoding nucleotide sequences selected from rat ADAM6, mouse ADAM6a and mouse ADAM6b-encoding nucleotide sequences. Optionally, the genome comprises 2 copies of ADAM6a and one copy (heterozygous) of ADAM6b; or one copy of ADAM6a and 2 copies of ADAM6b.

In one example of the homozygous mouse or rat of the invention, the genome comprises one or more transgenic light chain loci each comprising one or more human light chain V gene segments and one or more light chain J gene segments operably connected upstream of a light chain constant region (eg, an endogenous mouse or rat C kappa constant region).

### Inactivation of Endogenous Antibody Chain Expression by Translocation

In one configuration, the invention provides:-
A non-human vertebrate (optionally a mouse or rat) or non-human vertebrate cell (optionally a mouse or rat cell) having a genome that
(i) comprises one or more transgenic antibody loci capable of expressing antibodies comprising human variable regions (optionally following antibody gene rearrangement); and
(ii) is inactivated for endogenous antibody expression;
   wherein
(iii) endogenous variable region gene segments have been translocated to a chromosomal species (eg, chromosome 15) that does not contain antibody variable region gene segments in wild-type vertebrates of said non-human type, whereby endogenous antibody expression is inactivated.

The vertebrate can be any non-human vertebrate species disclosed herein. The transgenic antibody loci can be according to any one disclosed herein. The cell can be an ES cell, IPS cell, B-cell or any other non-human vertebrate cell disclosed herein.

In an example, the endogenous variable region gene segments have been translocated to a chromosomal species (eg, chromosome 15) that does not contain antibody variable region gene segments in wild-type vertebrates of said non-human type by translocation in an ancestor cell (eg, an ES cell) from which the vertebrate or cell of the invention is derived.

The invention also provides:-
A mouse or mouse cell having a genome that
(i) comprises one or more transgenic antibody loci capable of expressing antibodies comprising human variable regions (optionally following antibody gene rearrangement); and
(ii) is inactivated for endogenous mouse antibody expression;
   wherein
(iii) a plurality of endogenous mouse variable region gene segments are absent from chromosomes 12 in the genome, but are present in germline configuration (with respect to each other) on one or more chromosomes other than chromosomes 12 (eg, the gene segments are on chromosome 15), whereby endogenous mouse antibody expression is inactivated.

Furthermore, the invention provides:-
A rat or rat cell having a genome that
(i) comprises one or more transgenic antibody loci capable of expressing antibodies comprising human variable regions (optionally following antibody gene rearrangement); and
(ii) is inactivated for endogenous rat antibody expression;
   wherein
(iii) a plurality of endogenous rat variable region gene segments are absent from chromosomes 6 in the genome, but are present in germline configuration (with respect to each other) on one or more chromosomes other than chromosomes 6 (eg, the gene segments are on chromosome 15), whereby endogenous rat antibody expression is inactivated.

When the invention relates to a cell, such as an ES cell, inactivation of endogenous antibody expression relates to the inability of a differentiated antibody-producing progeny cell or non-human vertebrate to express endogenous antibodies, ie, antibodies whose variable regions are only of said non-human vertebrate type (eg, mouse or rat antibodies) and not human variable regions. Thus, in the present invention, the vertebrate, mouse, rat only expresses transgenic antibodies that comprise human variable regions and does not (or not substantially) express endogenous antibodies. The transgenic antibody loci may, in an example, undergo rearrangement *in vivo,* eg, following immunisation of the vertebrate, mouse or rat with a predetermined antigen. Following rearrangement, the organism is capable of expressing antibody chains from said rearranged loci, which chains form antibodies comprising human variable regions.

In an embodiment, the vertebrate, mouse, rat or cell genome comprises a transgenic antibody heavy chain locus (in heterozygous or homozygous state), the locus comprising one or more human VH gene segments, one or more human D gene segments and one or more human JH gene segments operably connected upstream of a non-human vertebrate (eg, mouse or rat) constant region (optionally Cmu and/or Cgamma); and said endogenous variable region gene segments are selected from endogenous
(a) VH;
(b) D;
(c) JH;
(d) VH and D;
(e) D and JH; and
(f) VH, D and JH.

In (a) to (f), intervening sequences between gene segments can be included.

In an example of this embodiment, the constant region is an endogenous constant region, eg, endogenous Cmu and/or Cgamma, such as endogenous mouse Cmu and/or endogenous mouse Cgamma.

In an embodiment, the vertebrate, mouse, rat or cell genome comprises expressible endogenous ADAM6 gene(s) or ADAM6-encoding nucleotide sequence(s).

In an embodiment, the vertebrate, mouse, rat or cell is a male vertebrate, mouse, rat or cell, eg, one whose genome comprises endogenous ADAM6 gene(s).

In one embodiment, substantially the entire endogenous VDJ (or part thereof including ADAM6-encoding nucleotide sequence(s)) is deleted from the chromosome by translocation to a different chromosome species. For example, the different chromosome is chromosome 15. Translocation between chromosomes 12 and 15 in a mouse, for example, is desirable since it is known from published observations that translocation between the heavy chain locus on chromosome 12 and c-*myc* on chromosome 15 is possible (see, eg, Science 24 December 1982: Vol. 218 no. 4579 pp. 1319-1321; "Mouse c-myc oncogene is located on chromosome 15 and translocated to chromosome 12 in plasmacytomas"; Crews *et al*)*.* Thus, in one example where the vertebrate is a mouse, the endogenous VDJ (or part thereof) is deleted from chromosome 12 by translocation to a chromosome 15. In another example, where the vertebrate is a rat, the endogenous VDJ (or part thereof) is deleted from chromosome 6 by translocation to a chromosome 15. Thus, in the final fertile mouse or progeny of the invention, endogenous heavy chain expression is inactivated by translocation of at least part of the endogenous heavy chain loci VDJ to a non-wild-type chromosome (ie, not a chromosome 12). Thus, in the final fertile rat or progeny of the invention, endogenous heavy chain expression is inactivated by translocation of at least part of the endogenous heavy chain loci VDJ to a non-wild-type chromosome (ie, not a chromosome 6). In this case, the translocated endogenous VDJ (or part) is retained in the animal's genome, but is rendered non-functional for endogenous heavy chain expression. This is advantageous because the endogenous ADAM6 genes are deleted from the wild-type chromosomal location to effect inactivation, but are then inserted into the genome elsewhere on an entirely different chromosomal species (ie, one not harbouring an antibody heavy chain locus) by translocation in a way that enables the inserted endogenous ADAM6 genes to function (and thus give fertility in downstream animals) without re-activating endogenous heavy chain expression. Thus, translocation enables inactivation with concomitant retention of endogenous, wild-type ADAM6 genes to provide for fertility in resultant animals. This perfectly tailors the ADAM6 genes to the animal's genome (since it is the endogenous sequence), and also in one embodiment enables transfer of each inserted endogenous ADAM6 genes together with its endogenous promoter (and any other control elements such as enhancers). Thus, in one embodiment inactivation is carried out by the deletion of a chromosomal sequence (eg, sequence of chromosome 12 in a mouse or 6 in a rat) comprising one or more ADAM6 genes including respective promoter(s) and this is inserted by translocation to a chromosome that does not comprise a heavy chain locus (eg, in a mouse a chromosome other than a chromosome 12; in a rat a chromosome other than a chromosome 6). This can be achieved, for example by translocating at least the DNA immediately flanked by the 3' most endogenous VH gene segment and the 5' most endogenous D segment. In one example, where the non-human vertebrate is a mouse, the translocated DNA comprises or consists of DNA from mouse V_{H}5-1 to D1-1 gene segments. In an embodiment, the entire endogenous VD region is translocated; in another embodiment the entire VDJ region is translocated, in either case this will also translocate the embedded endogenous ADAM6 genes.

Thus, the invention provides:-
A method of making a non-human vertebrate cell (optionally a mouse or rat cell) or a non-human vertebrate (eg, a mouse or rat), the method comprising
(i) inserting into a non-human ES cell genome one or more transgenic antibody loci comprising human variable region gene segments; and
(ii) inactivating endogenous antibody expression by translocating endogenous variable region gene segments (eg, an entire endogenous heavy chain VDJ region) to a chromosomal species (eg, chromosome 15) that does not contain antibody variable region gene segments in wild-type vertebrates of said non-human type;
whereby a non-human vertebrate ES cell is produced that is capable of giving rise to a progeny cell (eg, a B-cell or hybridoma) in which endogenous antibody expression is inactivated and wherein the progeny is capable of expressing antibodies comprising human variable regions; and
(iii) Optionally differentiating said ES cell into said progeny cell or a non-human vertebrate (eg, mouse or rat) comprising said progeny cell.

In an example, an entire (or substantially entire) endogenous heavy chain VDJ region including intervening sequences in germline configuration is translocated. Optionally, the genome of the cell/vertebrate is homozygous for this translocation. Alternatively or additionally, a light chain VJ region is translocated, eg, an entire (or substantially entire) endogenous light chain (eg, kappa) VJ region including intervening sequences in germline configuration is translocated.

Non-human vertebrates of the invention are useful for generating antibodies following immunisation with a target antigen or epitope of interest. Usefully, the antibodies that are generated have human heavy chain (and optionally also light chain) variable regions. The heavy chain (and optionally light chain) constant regions are of the non-human species, eg, endogenous to the animal, this allows for harnessing of the endogenous antibody expression and B-cell development control mechanisms, thereby enhancing antibody generation. After isolation following antigen immunisation, a selected antibody can be formatted by swapping the constant region for a human constant region by conventional techniques to increase compatibility for human administration.

The antibodies isolated from the animals of the invention (or derivative antibodies) be of any format provided that they comprise human heavy chain variable regions. For example, the present invention is applicable to of 4-chain antibodies, where the antibodies each contain 2 heavy chains and 2 light chains. Alternatively, the invention can be applied to H2 antibodies (heavy chain antibodies) bearing human V regions and which are devoid of CH1 and light chains (equivalent in respects to Camelid H2 antibodies: see, eg, Nature. 1993 Jun 3;363(6428):446-8; Naturally occurring antibodies devoid of light chains; Hamers-Casterman C, Atarhouch T, Muyldermans S, Robinson G, Hamers C, Songa EB, Bendahman N, Hamers R). These antibodies function to specifically bind antigen, such antibodies being akin to those found in the blood of *Camelidae* (eg, llamas, camels, alpacas). Such antibodies with human VH pairs can be synthetically produced to provide therapeutic and prophylactic medicaments (eg, see WO1994004678, WO2004041862, WO2004041863). Transgenic mice also can produce such heavy chain antibodies and the *in vivo* production of the antibodies allows the mouse's immune system to select for human VH-VH pairings, sometimes selecting for such pairings in which mutations have been introduced *in vivo* by the mouse to accommodate the pairing (WO2010109165A2). Thus, in an embodiment of the present invention, the heavy chain transgene is devoid of a CH1 gene segment and the genome comprises no functional antibody light chain locus. Alternatively, the test antibody is an antibody fragment, eg, Fab or Fab₂, which comprises a constant region and human heavy chain variable regions.

The skilled person will be familiar with routine methods and protocols for immunising with antigen, eg, using prime and boost immunisation protocols. A suitable protocol is RIMMS (see Hybridoma 1997 Aug;16(4):381-9; "Rapid development of affinity matured monoclonal antibodies using RIMMS"; Kilpatrick *et al*)*.* For immunisation of a vertebrate of the invention a suitable human target or epitope can be from any suitable source, eg, obtained by cloning the DNA from a blood or tissue sample of a human donor.

Throughout this text, and with application to any configuration, aspect, embodiment or example of the invention, the term "endogenous" (eg, endogenous constant region) in relation to a non-human vertebrate or cell, element or feature thereof (eg, "endogenous ADAM6" or "endogenous constant region") indicates that the element is a type of element that is normally found in the vertebrate or cell of that non-human species or strain (as opposed to an exogenous constant region, ADAM6 or other element whose sequence is not normally found in such a vertebrate or cell).

In one example, each mouse or ES cell is one having a 129 mouse genetic background. In one example, the mouse or ES cell has an AB2.1 mouse genetic background. In another example, the mouse or ES cell has a genetic background of a mouse strain selected from 129, C57BL/6N, C57BL/6J, JM8, AB2.1, AB2.2, 129S5 or 129Sv.

An antibody isolated from a vertebrate of the invention can be subsequently derivatised, eg, by the addition (such as by chemical conjugation) of a label or toxin, PEG or other moiety, to make a pharmaceutical product. Derivatisation is useful, for example, when it is desirable to add an additional functionality to the drug to be developed from the antibody. For example, for cancer indications it may be desirable to add additional moieties that assist in cell-killing. In another embodiment, the variable regions of the antibody isolated from the vertebrate are affinity matured *in vivo* or *in vitro* (eg, by phage display, ribosome display, yeast display, *etc*). In another embodiment, the constant regions of the antibody isolated from the vertebrate are mutated *in vivo* or *in vitro* (eg, by random or directed, specific mutation and optional selection by phage display, ribosome display, yeast display, *etc*)*.* The constant region may be mutated to ablate or enhance Fc function (eg, ADCC).

In one embodiment, the genome of the final vertebrate comprises one or more light chain antibody loci comprising human VJ gene segments, eg, as described in any of WO2011004192, US7501552, US6673986, US6130364, WO2009076464 and US6586251, the disclosures of which are incorporated herein by reference in their entirety. In one example, the final vertebrate comprises
(a) Heavy chain loci, each comprising one or more human heavy chain V gene segments, one or more human heavy chain D gene segments and one or more human heavy chain JH gene segments upstream of an endogenous non-human vertebrate (eg, endogenous mouse or rat) constant region (eg, Cmu and/or Cgamma);
(b) A kappa light chain locus (optionally in homozygous state) comprising one or more human kappa chain V gene segments, and one or more human kappa chain Jk gene segments upstream of an endogenous non-human vertebrate (eg, endogenous mouse or rat) kappa constant region; and optionally
(c) A lambda light chain locus (optionally in homozygous state) comprising one or more human lambda chain V gene segments, and one or more human lambda chain Jλ gene segments upstream of a lambda constant region; and
(d) Wherein the vertebrate is capable of producing chimaeric antibodies following rearrangement of said loci and immunisation with an antigen.

As is conventional in the art, there are provided methods for generating IPS cells. For example, mouse embryo fibroblasts can be generated from a mouse embryo and then IPS cells generated using any standard technique. For example, reference is made to Proc Natl Acad Sci; 2011 Oct 11; "Rapid and efficient reprogramming of somatic cells to induced pluripotent stem cells by retinoic acid receptor gamma and liver receptor homolog 1"; Wang *et al,* the disclosure of which is incorporated herein by reference. Other standard IPS-generating techniques can be used.

In one embodiment of any aspect of the invention, when an IPS cells is used, the IPS cell is a mouse embryonic fibroblast cell.

Human DNA (eg, as a source of heavy and/or light chain gene segments) is readily obtainable from commercial and academic libraries, eg, Bacterial Artificial Chromosome (BAC) libraries containing human DNA. Examples are the Human RPCI-11 and -13 libraries (Osoegawa *et al,* 2001 - see below; http://bacpac.med.buffalo.edu/11framehmale.htm) and also the "CalTech" Human BAC libraries (CalTech Libraries A, B, C and/or D, http://www.tree.caltech.edu/lib status.html).

### CalTech human BAC library D:

See: http://www.ncbi.nlm.nih.gov/clone/library/genomic/16/
The Hiroaki Shizuya laboratory at the California Institute of Technology has developed three distinct human BAC libraries (obtainable from Open Biosystems). The Cal Tech B (CTB) and Cal Tech C (CTC) libraries together represent a genomic coverage of 15X. The Cal Tech D (CTD) library represents a 17X coverage of the human genome. Whole collections as well as individual clones are available. Detailed information on the construction of the libraries can be found at http://informa.bio.caltech.edu/idx www tree.html.

### Library Summary

Library Name: CalTech human BAC library D
Library Abbreviation: CTD
Organism: Homo sapiens
Distributors: Invitrogen, Open Biosystems
Vector type(s): BAC
# clones Clone DB: 226,848
# end sequences Clone DB: 403,688
# insert sequences Clone DB: 3,153
# clones with both ends sequenced: 153,035

### Library Details

| **DNA Source:** | **Sex** | **Cell type** | |
|---|---|---|---|
| | male | Sperm | |

| **Library Construction** | **Library segment** | **Vector Name** | **Vector Cloning Site(s)** |
|---|---|---|---|
| | 1 | pBeloBACII | Hindlll |
| | 2-5 | pBeloBACII | EcoRI |

| **Library Statistics** | **Library segment** | **Avg Insert (kb)** | **Plate Range(s)** |
|---|---|---|---|
| | 1 | 129 | 2001 to 2423 |
| | 2 | 202 | 2501 to 2565 |
| | 3 | 182 | 2566 to 2671 |
| | 4 | 142 | 3000 to 3253 |
| | 5 | 166 | 3254 to 4869 |

### RPCI-11 BACs

### References

- Osoegawa K, Mammoser AG, Wu C, Frengen E, Zeng C, Catanese JJ, de Jong PJ; Genome Res. 2001 Mar;11(3):483-96; "A bacterial artificial chromosome library for sequencing the complete human genome";
- Osoegawa, K., Woon, P.Y., Zhao, B., Frengen, E., Tateno, M., Catanese, J.J, and de Jong, P.J. (1998); "An Improved Approach for Construction of Bacterial Artificial Chromosome Libraries"; Genomics 52, 1-8;
- http://bacpac.chori.org/hmale11.htm, which describes the BACs as follows

### BAC Availability

The RP11 BACs are available for purchase from Invitrogen (see http://tools.invitrogen.com/content/sfs/manuals/bac clones man.pdf).

Vectors, such as BACs or PACs, can be manipulated *in vitro* by standard Molecular Biology techniques, for example recombineering (see http://www.genebridges.com; EP129142 and EP1204740). For example, recombineering can be used to create vectors in which a nucleotide sequence coding for human DNA of interest is flanked by one or more sequences, such as homology arms or site-specific recombination sites (eg, lox, frt or rox). The homology arms are, in one embodiment, homologous to, or identical to, stretches of DNA from the genome of the non-human vertebrate to be used to generate the vertebrate. Vectors created in this way are useful for performing homologous recombination (see, eg, US6638768, the disclosure of which is incorporated herein by reference) in a method of precisely inserting the human DNA into the non-human vertebrate genome (eg, to precisely replace the orthologous or homologous DNA in the vertebrate genome).

Other useful DNA- and genome-manipulation techniques are readily available to the skilled person, including technologies described in US6461818 (Baylor College of Medicine), US6586251 (Regeneron) and WO2011044050 (eg, see Examples).

Techniques for constructing non-human vertebrates and vertebrate cells whose genomes comprise a transgene, eg, a transgenic antibody locus containing human V, J and optionally D regions are well known in the art. For example, reference is made to WO2011004192, US7501552, US6673986, US6130364, WO2009/076464 and US6586251, the disclosures of which are incorporated herein by reference in their entirety.

All nucleotide coordinates for the mouse are from NCBI m37, April 2007 ENSEMBL Release 55.37h for the mouse C57BL/6J strain. Human nucleotides are from GRCh37, Feb 2009 ENSEMBL Release 55.37 and rat from RGSC 3.4 Dec 2004 ENSEMBL release 55.34w.

In one embodiment of a vertebrate of the invention, the vertebrate is a mammal, eg, a rodent. In one embodiment of a vertebrate of the invention, the vertebrate is a mouse, rat, rabbit, Camelid (eg, a llama, alpaca or camel) or shark.

In one aspect the transgenic antibody loci comprise human V, D and/or J coding regions placed under control of the host regulatory sequences or other (non-human, non-host) sequences. In one aspect reference to human V, D and/or J coding regions includes both human introns and exons, or in another aspect simply exons and no introns, which may be in the form of cDNA.

Alternatively it is possible to use recombineering, or other recombinant DNA technologies, to insert a non human-vertebrate (e.g. mouse) promoter or other control region, such as a promoter for a V region, into a BAC containing a human Ig region. The recombineering step then places a portion of human DNA under control of the mouse promoter or other control region.

The invention also relates to a cell line (eg, ES or IPS cell line) which is grown from or otherwise derived from cells or a vertebrate as described herein, including an immortalised cell line. The cell line may be immortalised by fusion to a tumour cell to provide an antibody producing cell and cell line, or be made by direct cellular immortalisation.

In one aspect the non-human vertebrate of any configuration of the invention is able to generate a diversity of at least 1 × 10⁶ different functional chimaeric antibody sequence combinations.

Optionally in any configuration of the invention the constant region is endogenous to the vertebrate and optionally comprises an endogenous switch. In one embodiment, the constant region comprises a Cgamma (CY) region and/or a Smu (Sµ) switch. Switch sequences are known in the art, for example, see Nikaido et al, Nature 292: 845-848 (1981) and also WO2011004192, US7501552, US6673986, US6130364, WO2009/076464 and US6586251, eg, SEQ ID NOs: 9-24 disclosed in US7501552. Optionally the constant region comprises an endogenous S gamma switch and/or an endogenous Smu switch.

In one optional aspect where the Vertebrate is a mouse, the insertion of the human antibody gene DNA, such as the human VDJ region is targeted to the region between the J4 exon and the Cµ locus in the mouse genome IgH locus, and in one aspect is inserted between coordinates 114,667,090 and 114,665,190, suitably at coordinate 114,667,091. In one aspect the insertion of human light chain kappa VJ is targeted into mouse chromosome 6 between coordinates 70,673,899 and 70,675,515, suitably at position 70,674,734, or an equivalent position in the lambda mouse locus on chromosome 16.

Reference to location of the variable region upstream of the non-human vertebrate constant region means that there is a suitable relative location of the two antibody portions, variable and constant, to allow the variable and constant regions to form a chimaeric antibody or antibody chain *in vivo* in the vertebrate. Thus, the inserted human antibody DNA and host constant region are in operable connection with one another for antibody or antibody chain production.

In one aspect the inserted human antibody DNA is capable of being expressed with different host constant regions through isotype switching. In one aspect isotype switching does not require or involve trans switching. Insertion of the human variable region DNA on the same chromosome as the relevant host constant region means that there is no need for trans-switching to produce isotype switching.

In the present invention, optionally at least one non-human vertebrate enhancer or other control sequence, such as a switch region, is maintained in functional arrangement with the non-human vertebrate constant region, such that the effect of the enhancer or other control sequence, as seen in the host vertebrate, is exerted in whole or in part in the transgenic animal. This approach is designed to allow the full diversity of the human locus to be sampled, to allow the same high expression levels that would be achieved by non-human vertebrate control sequences such as enhancers, and is such that signalling in the B-cell, for example isotype switching using switch recombination sites, would still use non-human vertebrate sequences.

A non-human vertebrate having such a genome would produce chimaeric antibodies with human variable and non-human vertebrate constant regions, but these are readily humanized, for example in a cloning step that replaces the mouse constant regions for corresponding human constant regions.

In one aspect the inserted human IgH VDJ region comprises, in germline configuration, all of the V, D and J regions and intervening sequences from a human. Optionally, non-functional V and/or D and/or J gene segments are omitted. For example, VH which are inverted or are pseudogenes may be omitted.

In one aspect 800-1000kb of the human IgH VDJ region is inserted into the non-human vertebrate IgH locus, and in one aspect a 940, 950 or 960 kb fragment is inserted. Suitably this includes bases 105,400,051 to 106,368,585 from human chromosome 14 (all coordinates refer to NCBI36 for the human genome, ENSEMBL Release 54 and NCBIM37 for the mouse genome, relating to mouse strain C57BL/6J).

In one aspect the inserted IgH human fragment consists of bases 105,400,051 to 106,368,585 from chromosome 14. In one aspect the inserted human heavy chain DNA, such as DNA consisting of bases 105,400,051 to 106,368,585 from chromosome 14, is inserted into mouse chromosome 12 between the end of the mouse J4 region and the Eµ region, suitably between coordinates 114,667,091 and 114,665,190, suitably at coordinate 114,667,091.

In one aspect the inserted human kappa VJ region comprises, in germline configuration, all of the V and J regions and intervening sequences from a human. Optionally, non-functional V and/or J gene segments are omitted.

Suitably this includes bases 88,940,356 to 89,857,000 from human chromosome 2, suitably approximately 917kb. In a further aspect the light chain VJ insert may comprise only the proximal clusters of V segments and J segments. Such an insert would be of approximately 473 kb.

In one aspect the human light chain kappa DNA, such as the human IgK fragment of bases 88,940,356 to 89,857,000 from human chromosome 2, is suitably inserted into mouse chromosome 6 between coordinates 70,673,899 and 70,675,515, suitably at position 70,674,734.

In one aspect the human lambda VJ region comprises, in germline configuration, all of the V and J regions and intervening sequences from a human. Suitably this includes analogous bases to those selected for the kappa fragment, from human chromosome 2. Optionally, non-functional V and/or J gene segments are omitted.

All specific human antibody fragments described herein may vary in length, and may for example be longer or shorter than defined as above, such as 500 bases, 1KB, 2K, 3K, 4K, 5KB, 10 KB, 20KB, 30KB, 40KB or 50KB or more, which suitably comprise all or part of the human V(D)J region, whilst preferably retaining the requirement for the final insert to comprise human genetic material encoding the complete heavy chain region and light chain region, as appropriate, as described herein.

In one aspect the 3' end of the last inserted human antibody sequence, generally the last human J sequence, is inserted less than 2kb, preferably less than 1KB from the human/non-human vertebrate (eg, human/mouse or human/rat) join region.

Optionally, the genome is homozygous at the heavy chain locus and one, or both of Igλ and Igκ loci.

In another aspect the genome may be heterozygous at one or more of the light chain antibody loci, such as heterozygous for DNA encoding a chimaeric antibody chain and native (host cell) antibody chain. In one aspect the genome may be heterozygous for DNA capable of encoding 2 different antibody chains encoded by immunoglobulin transgenes of the invention, for example, comprising 2 different chimaeric heavy chains or 2 different chimaeric light chains.

In one embodiment in any configuration of the invention, the genome of the vertebrate has been modified to prevent or reduce the expression of fully-endogenous antibody. Examples of suitable techniques for doing this can be found in WO2011004192, US7501552, US6673986, US6130364, WO2009/076464, EP1399559 and US6586251, the disclosures of which are incorporated herein by reference. In one embodiment, the non-human vertebrate VDJ region of the endogenous heavy chain immunoglobulin locus, and optionally VJ region of the endogenous light chain immunoglobulin loci (lambda and/or kappa loci), have been inactivated. For example, all or part of the non-human vertebrate VDJ region is inactivated by inversion in the endogenous heavy chain immunoglobulin locus of the mammal, optionally with the inverted region being moved upstream or downstream of the endogenous Ig locus. For example, all or part of the non-human vertebrate VJ region is inactivated by inversion in the endogenous kappa chain immunoglobulin locus of the mammal, optionally with the inverted region being moved upstream or downstream of the endogenous Ig locus. For example, all or part of the non-human vertebrate VJ region is inactivated by inversion in the endogenous lambda chain immunoglobulin locus of the mammal, optionally with the inverted region being moved upstream or downstream of the endogenous Ig locus. In one embodiment the endogenous heavy chain locus is inactivated in this way as is one or both of the endogenous kappa and lambda loci.

Additionally or alternatively, the vertebrate has been generated in a genetic background which prevents the production of mature host Band T lymphocytes, optionally a RAG-1-deficient and/or RAG-2 deficient background. See US5859301 for techniques of generating RAG-1 deficient animals.

In one embodiment in any configuration of the invention, the human V, J and optional D regions are provided by all or part of the human IgH locus; optionally wherein said all or part of the IgH locus includes substantially the full human repertoire of IgH V, D and J regions and intervening sequences. A suitable part of the human IgH locus is disclosed in WO2011004192. In one embodiment, the human IgH part includes (or optionally consists of) bases 105,400,051 to 106,368,585 from human chromosome 14 (coordinates from NCBI36). Additionally or alternatively, optionally wherein the vertebrate is a mouse or the cell is a mouse cell, the human V, J and optional D regions are inserted into mouse chromosome 12 at a position corresponding to a position between coordinates 114,667,091 and 114,665,190, optionally at coordinate 114,667,091 (coordinates from NCBIM37, relating to mouse strain C57BL/6J).

In one embodiment of any configuration of a vertebrate or cell (line) of the invention when the vertebrate is a mouse, (i) each transgenic heavy chain locus of the mouse genome comprises a constant region comprising a mouse or rat 5µ switch and optionally a mouse Cµ region. For example the constant region is provided by the constant region endogenous to the mouse (mouse cell), eg, by inserting human V(D)J region sequences into operable linkage with the endogenous constant region of a mouse genome or mouse cell genome.

In one embodiment of any configuration of a vertebrate or cell (line) of the invention when the Vertebrate is a rat, (i) each transgenic heavy chain locus of the rat genome comprises a constant region comprising a mouse or rat 5µ switch and optionally a rat Cµ region. For example the constant region is provided by the constant region endogenous to the rat, eg, by inserting human V(D)J region sequences into operable linkage with the endogenous constant region of a rat genome or rat cell genome.

In one embodiment of any configuration of a vertebrate or cell (line) of the invention the genome comprises a lambda antibody transgene comprising all or part of the human Igλ locus including at least one human Jλ region and at least one human Cλ region, optionally C_{λ}6 and/or C_{λ}7. Optionally, the transgene comprises a plurality of human Jλ regions, optionally two or more of J_{λ}1, J_{λ}2, J_{λ}6 and J_{λ}7, optionally all of J_{λ}1, J_{λ}2, J_{λ}6 and J_{λ}7. The human lambda immunoglobulin locus comprises a unique gene architecture composed of serial J-C clusters. In order to take advantage of this feature, the invention in optional aspects employs one or more such human J-C clusters inoperable linkage with the constant region in the transgene, eg, where the constant region is endogenous to the non-human vertebrate or non-human vertebrate cell (line). Thus, optionally the transgene comprises at least one human J_{λ}-C_{λ} cluster, optionally at least J_{λ}7-C_{λ}7. The construction of such transgenes is facilitated by being able to use all or part of the human lambda locus such that the transgene comprises one or more J-C clusters in germline configuration, advantageously also including intervening sequences between clusters and/or between adjacent J and C regions in the human locus. This preserves any regulatory elements within the intervening sequences which may be involved in VJ and/or JC recombination and which may be recognised by AID (activation-induced deaminase) or AID homologues.

Where endogenous regulatory elements are involved in CSR (class-switch recombination) in the non-human vertebrate, these can be preserved by including in the transgene a constant region that is endogenous to the non-human vertebrate. In the first configuration of the invention, one can match this by using an AID or AID homologue that is endogenous to the vertebrate or a functional mutant thereof. Such design elements are advantageous for maximising the enzymatic spectrum for SHM (somatic hypermutation) and/or CSR and thus for maximising the potential for antibody diversity.

Optionally, the lambda transgene comprises a human Eλ enhancer. Optionally, the kappa transgene comprises a human Eκ enhancer. Optionally, the heavy chain transgene comprises a heavy chain human enhancer.

In one embodiment of any configuration of the invention the constant region of the or each antibody transgene is endogenous to the non-human vertebrate or derived from such a constant region. For example, the vertebrate is a mouse or the cell is a mouse cell and the constant region is endogenous to the mouse. For example, the vertebrate is a rat or the cell is a rat cell and the constant region is endogenous to the rat.

In one embodiment of any configuration of the invention each heavy chain transgene comprises a plurality human IgH V regions, a plurality of human D regions and a plurality of human J regions, optionally substantially the full human repertoire of IgH V, D and J regions.

In one embodiment of any configuration of the invention, for the vertebrate:-
(i) each heavy chain transgene comprises substantially the full human repertoire of IgH V, D and J regions; and
(ii) the vertebrate genome comprises substantially the full human repertoire of Igκ V and J regions and/or substantially the full human repertoire of Igλ V and J regions.

An aspect provides a B-cell, hybridoma or a stem cell, optionally an embryonic stem cell or haematopoietic stem cell, derived from a vertebrate according to any configuration of the invention. In one embodiment, the cell is a BALB/c, JM8 or AB2.1 or AB2.2 embryonic stem cell (see discussion of suitable cells, and in particular JM8 and AB2.1 cells, in WO2011004192, which disclosure is incorporated herein by reference).

In one aspect the ES cell is derived from the mouse BALB/c, C57BL/6N, C57BL/6J, 129S5 or 129Sv strain.

In one aspect the non-human vertebrate is a rodent, suitably a mouse, and cells (cell lines) of the invention, are rodent cells or ES cells, suitably mouse ES cells.

The ES cells of the present invention can be used to generate animals using techniques well known in the art, which comprise injection of the ES cell into a blastocyst followed by implantation of chimaeric blastocystys into females to produce offspring which can be bred and selected for homozygous recombinants having the required insertion. In one aspect the invention relates to a transgenic animal comprised of ES cell-derived tissue and host embryo derived tissue. In one aspect the invention relates to genetically-altered subsequent generation animals, which include animals having a homozygous recombinants for the VDJ and/or VJ regions.

An aspect provides a method of isolating an antibody or nucleotide sequence encoding said antibody, the method comprising
(a) immunising (see e.g. Harlow, E. & Lane, D. 1998, 5th edition, Antibodies: A Laboratory Manual, Cold Spring Harbor Lab. Press, Plainview, NY; and Pasqualini and Arap, Proceedings of the National Academy of Sciences (2004) 101:257-259) a vertebrate according to any configuration or aspect of the invention with a human target antigen such that the vertebrate produces antibodies; and
(b) isolating from the vertebrate an antibody that specifically binds to said antigen and/or a nucleotide sequence encoding at least the heavy and/or the light chain variable regions of said antibody;
optionally wherein the variable regions of said antibody are subsequently joined to a human constant region. Such joining can be effected by techniques readily available in the art, such as using conventional recombinant DNA and RNA technology as will be apparent to the skilled person. See e.g. Sambrook, J and Russell, D. (2001, 3'd edition) Molecular Cloning: A Laboratory Manual (Cold Spring Harbor Lab. Press, Plainview, NY).

Suitably an immunogenic amount of the human epitope or target antigen is delivered. The invention also relates to a method for detecting a human epitope or target antigen comprising detecting a test antibody produced as above with a secondary detection agent which recognises a portion of that antibody.

Isolation of the antibody in step (b) can be carried out using conventional antibody selection techniques, eg, panning for antibodies against antigen that has been immobilised on a solid support, optionally with iterative rounds at increasing stringency, as will be readily apparent to the skilled person.

As a further optional step, after step (b) the amino acid sequence of the heavy and/or the light chain variable regions of the antibody are mutated to improve affinity for binding to said antigen. Mutation can be generated by conventional techniques as will be readily apparent to the skilled person, eg, by error-prone PCR. Affinity can be determined by conventional techniques as will be readily apparent to the skilled person, eg, by surface plasmon resonance, eg, using Biacore^{™}.

Additionally or alternatively, as a further optional step, after step (b) the amino acid sequence of the heavy and/or the light chain variable regions of a test antibody are mutated to improve one or more biophysical characteristics of the antibody, eg, one or more of melting temperature, solution state (monomer or dimer), stability and expression (eg, in CHO or *E coli*)*.*

An aspect provides an antibody of the invention, optionally for use in medicine, eg, for treating and/or preventing a medical condition or disease in a patient, eg, a human.

An aspect provides a nucleotide sequence encoding an antibody of the invention, optionally wherein the nucleotide sequence is part of a vector. Suitable vectors will be readily apparent to the skilled person, eg, a conventional antibody expression vector comprising the nucleotide sequence together in operable linkage with one or more expression control elements.

An aspect provides a pharmaceutical composition comprising an antibody of the invention and a diluent, excipient or carrier, optionally wherein the composition is contained in an IV container (eg, and IV bag) or a container connected to an IV syringe.

An aspect provides the use of an antibody of the invention in the manufacture of a medicament for the treatment and/or prophylaxis of a disease or condition in a patient, eg a human.

In a further aspect the invention relates to humanised antibodies and antibody chains produced or assayed according to the present invention, both in chimaeric and fully humanised form, and use of said antibodies in medicine. The invention also relates to a pharmaceutical composition comprising such an antibody and a pharmaceutically acceptable carrier or other excipient.

Antibody chains containing human sequences, such as chimaeric human-non human antibody chains, are considered humanised herein by virtue of the presence of the human protein coding regions region. Fully human antibodies may be produced starting from DNA encoding a chimaeric antibody chain of the invention using standard techniques.

Methods for the generation of both monoclonal and polyclonal antibodies are well known in the art, and the present invention relates to both polyclonal and monoclonal antibodies of chimaeric or fully humanised antibodies produced in response to antigen challenge in non human-vertebrates of the present invention.

In a yet further aspect, chimaeric antibodies or antibody chains generated in the present invention may be manipulated, suitably at the DNA level, to generate molecules with antibody-like properties or structure, such as a human variable region from a heavy or light chain absent a constant region, for example a domain antibody; or a human variable region with any constant region from either heavy or light chain from the same or different species; or a human variable region with a non-naturally occurring constant region; or human variable region together with any other fusion partner. The invention relates to all such chimaeric antibody derivatives derived from chimaeric antibodies identified, isolated or assayed according to the present invention.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the feature in the context with which it is referred. The term "substantially" when referring to an amount, extent or feature (eg, "substantially identical" or "substantially the same") includes a disclosure of "identical" or "the same" respectively, and this provides basis for insertion of these precise terms into claims below.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

Any part of this disclosure may be read in combination with any other part of the disclosure, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. All such similar substitutes and modifications apparent to those skilled in the art are deemed to be within the spirit, scope and concept of the invention as defined by the appended claims.

The present invention is described in more detail in the following non limiting exemplification.

### EXAMPLES

The following examples will be useful for demonstrating the present invention.

### INACTIVATION OF ENDOGENOUS IGH GENES AND MAINTENANCE OF ADAM6 FUNCTION

### Example 1: Translocation

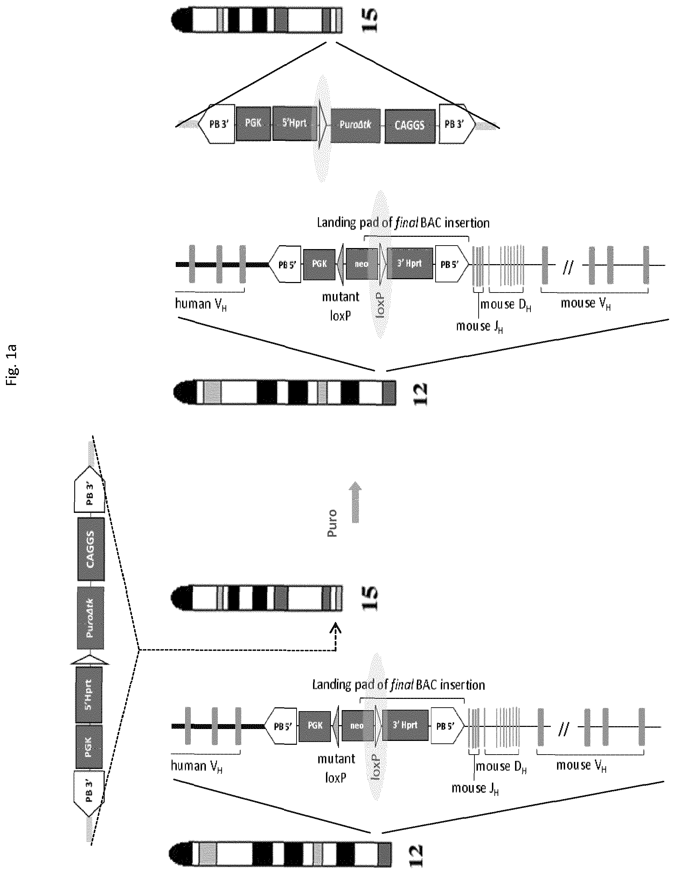

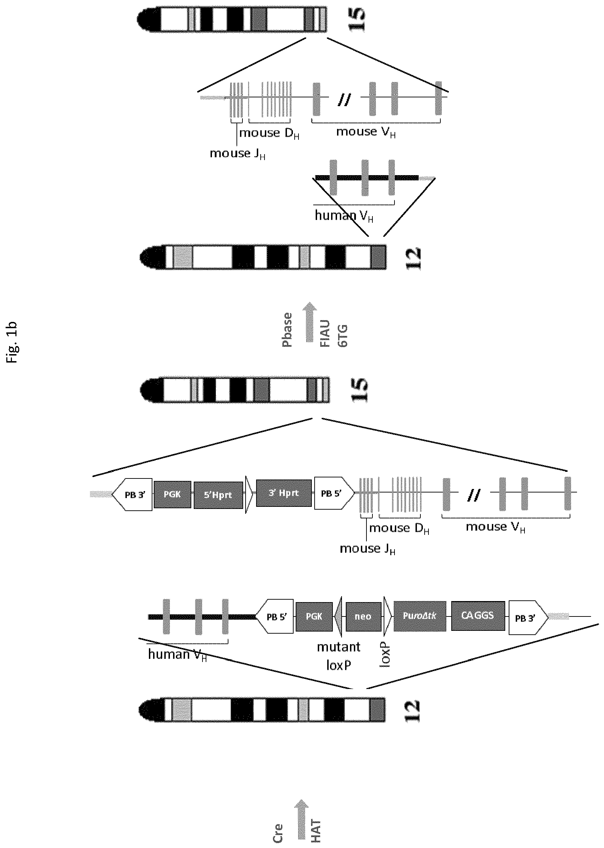

Reference is made to Fig 1a where chromosome 12 is shown harbouring a transgenic heavy chain locus. In the figure, the inserted human V_{H} gene segments are shown (but for clarity the human D and J_{H}, the mouse Emu enhancer and other J-C intronic elements, and also the constant region are not shown, but these lie downstream of the human V_{H} gene segments (ie, to the left of the V_{H}). Also shown is a loxP site on chromosome 12 between the human V_{H} and the mouse VDJ region (in this case the loxP being provided by a "landing pad"; see, eg, WO2011004192 the disclosure of which is incorporated herein by reference). A cassette, carrying a loxP site in the same direction to the loxP site in the landing pad, is targeted at the telomere region of a different chromosome from chromosome 12; in this case targeting is to chromosome 15 as shown in Fig 1a. A vector carrying a Cre recombinase gene is introduced into the cell. Following induction of Cre recombinase expression, the regions between the loxP sites and the telomeres are exchanged, which results in separation of the endogenous mouse V_{H}, D and J_{H} gene segments away from their enhancer and C region (Fig 1b) and thus inactivation of endogenous heavy chain. In this method, the upstream and downstream genome sequence of Adam6a and Adam6b which includes the associated regulatory elements of these two genes, is still retained intact. Thus functional, endogenous Adam6 genes are retained - in this case on chromosome 15 (Fig. 1b).

### Example 2: Deletion & Insertion of Adam 6 Genes

### Generation of Transgenic Antibody-Generating Mouse

A transgenic mouse is generated using ES cell technology and genetic manipulation to introduce human antibody heavy chain and kappa chain V, D and J segments operatively connected directly 5' of endogenous mouse heavy and kappa constant regions respectively. Mouse mu switch and mu constant and gamma regions are provided in the heavy chain transgenic locus thus produced. Endogenous, mouse heavy chain and kappa chain expression are inactivated; mouse lambda chain expression is typically 5% or less so inactivation is optional. The human antibody gene segments are introduced into a mouse ES cell using homologous recombination and/or recombinase mediated cassette exchange (RMCE) as is known in the art. Human DNA can be manipulated using BAC and recombineering technology as known in the art. BACs containing human antibody gene DNA is obtainable from Invitrogen. A suitable ES cell is a 129, AB2.1 or AB2.2 cell (obtainable from Baylor College of Medicine).

The transgenic ES cells are then implanted into a blastocyst from a foster mouse mother (eg, a 129 or C57BL/6N mouse strain). Heavy chain and kappa chain lines can be produced and crossed to provide an antibody-generating mouse bearing homozygous transgenic heavy and kappa chains with human variable regions (HK mouse).

Using a similar protocol, a lambda chain line is produced and by crossing a HKL mouse is generated bearing homozygous transgenic heavy, lambda and kappa chains with human variable regions.

Further guidance is disclosed in WO2011004192, US7501552, US6673986, US6130364, WO2009/076464 and US6586251, the disclosures of which are incorporated herein by reference in their entirety.

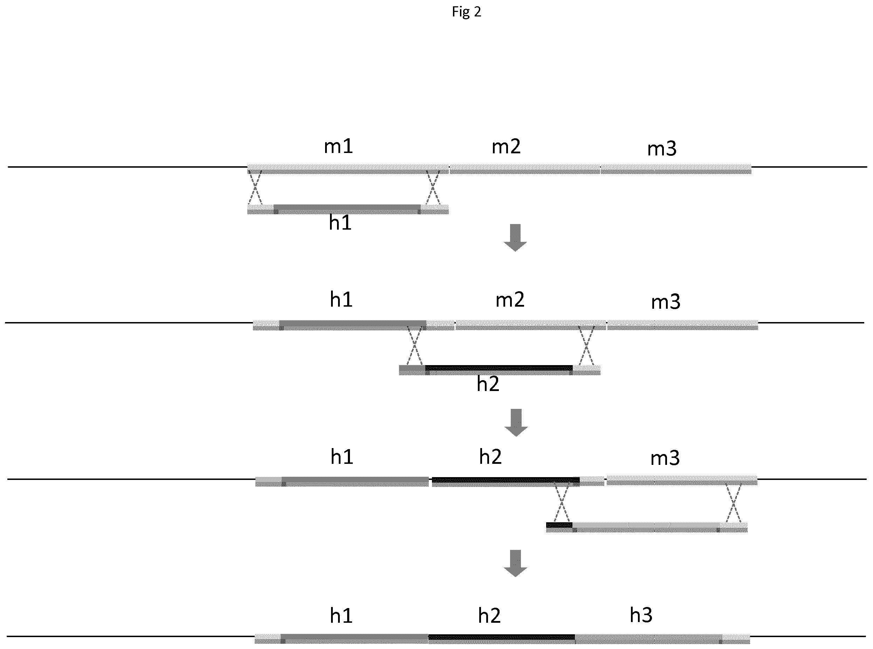

In order to introduce human heavy chain gene segments by homologous recombination, one or more BACs are generated using standard techniques such as recombineering. A large DNA targeting vector containing human genomic IGH gene segments (V_{H}s, Ds and J_{H}s), a selection marker and two flanking recombination arms (5' and 3') homologous to the endogenous IGH sequence is constructed by BAC modification (Fig 2; h1= first BAC containing human gene segments; m1= homologous region of the mouse VDJ region; and so on for h1, h2, m2 and m3). The large targeting vector is introduced into mouse ES cells by electroporation. The targeted ES cells are selected by drugs or other marker sorting for the selection marker as is conventional. The correct targeting by homologous recombination is further confirmed by either quantitative or qualitative PCR-based methods. The correctly targeted locus results in replacement of endogenous genomic DNA flanked by those two homologous recombination arms, in which this section of the endogenous locus is replaced with the human genomic IGH gene segments and a selection marker.

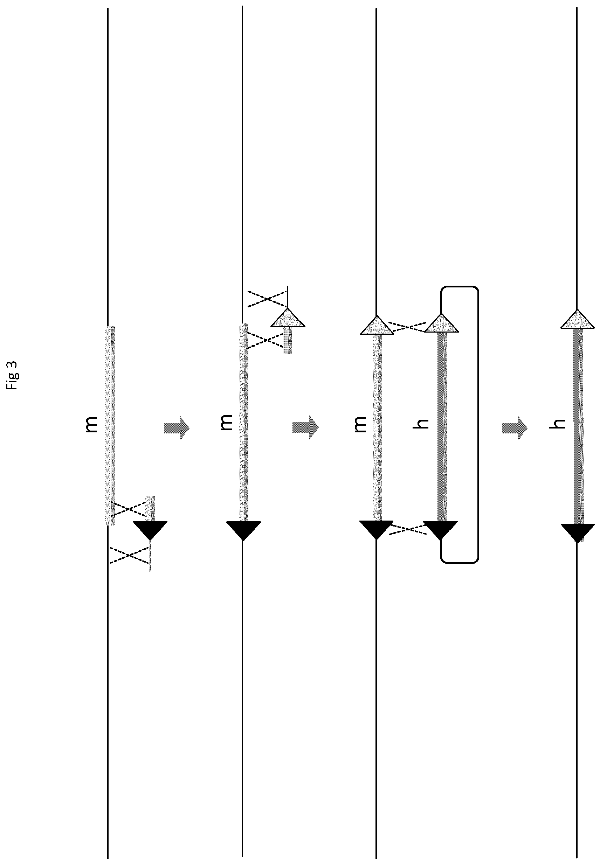

The human antibody heavy chain gene segments ("h" in Fig 3) can also be inserted using standard recombinase-mediated genomic replacement (Fig. 3). In such an approach, one loxP site and a mutant loxP site (such as Iox511) are sequentially targeted into the mouse IGH locus. A large DNA targeting vector containing human genomic IGH gene segments (V_{H}s, Ds and J_{H}s) and a selection marker, flanked by one loxP site and another copy of the mutant loxP is constructed by BAC modification. The large targeting vector is co-electroporated with a Cre-expressing vector into ES cells. The correct targeting is further confirmed by either quantitative or qualitative PCR-based methods. The correctly targeted locus results in the replacement of endogenous antibody locus genomic DNA flanked by those two lox sites, in which this section of the endogenous locus ("m" in Fig 3) is replaced with the human genomic IGH gene segments and a selection marker. In this process, endogenous Adam6 genes are also deleted.

During these two replacement processes (replacement by homolgous recombination or RMGR), the endogenous mouse Adam6 genes between the V_{H}5-1 and D1-1 gene segments are deleted. The genomic DNAs containing the Adam6 exons (Adam6a-2507 bp; Adam6b-2271 bp) as well as at least 5 kb upstream and 5 kb downstream sequences for each of them are inserted into mouse genome by either targeted or random insertion in ES cells or zygotes to rescue the male fertility of such Adam6-deleted mice as per Example 3.

### Example 3: Approaches to insert Adam6 genes into genome after endogenous IGH deletion

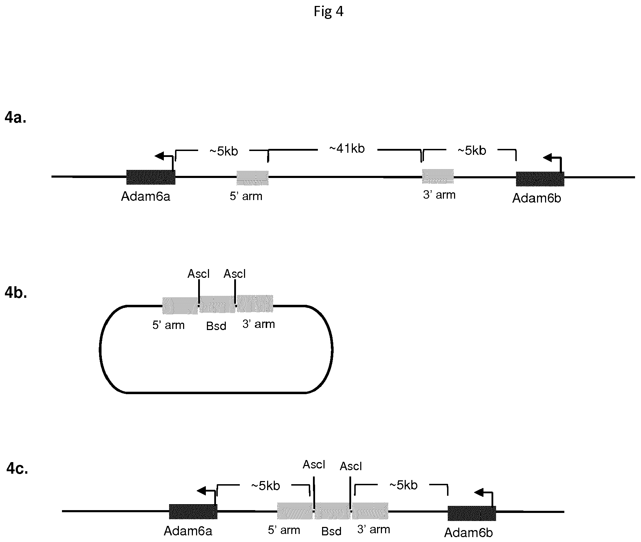

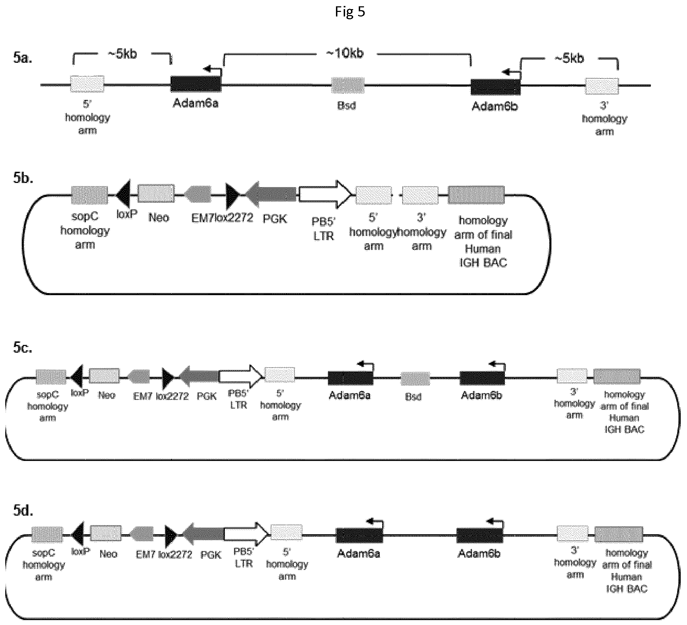

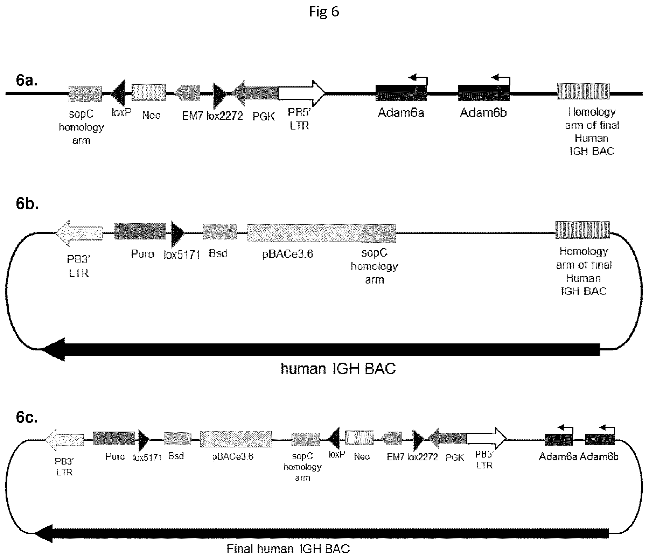

The mouse Adam6a (Chromosome 12: coordinates 114777119-114789625) and Adam6b (Chromosome 12: coordinates 114722756-114735229) genomic DNA is retrieved from a bacterial artificial chromosome (BAC), RP23-393F3 (Invitrogen). The ES-cell targeting vector is generated by the following steps.
1. The sequence between mouse Adam6a and Adam6b is deleted by a positive selection marker cassette.
   a. 5' arm which is located at ~ 5kb upstream of Adam6a and 3' arm which is located at ~5kb downstream of Adam6b gene are created by PCR using RP23-393F3 as a template. Both homology arms are between 200bp to 300bp, then the two homology arms are cloned into a plasmid based on pBlueScript II SK(+) and that contains a positive selection marker Blasticidin (Bsd) which flanked by two Asci sites, to build a deletion vector (Fig. 4a and 4b).
      5' Arm: 
5'-tatgttgatggatttccatatattaaaccatccctgcatccctgggatgaagcctacttggtcatgataga
cgattgttttgatgtgttcttggattcagttagtgagaaatatattgagtatttttacatcgatattcataaggg
aaattggtctgaagttctctttctttgttgggtctttatgtggtttagttatca-3'
      3' Arm: 
5'- tgattccaccagaggttcttttatccttgagaagagtttttgctatcctaggttttttgttattccacatgaatttg
cagattgctctttctaattccttgaagaattgagttggaatttgatggggattgcattaaatctgtagattccttttgg
caagacagccatttttacaatgttaatcctgccaatccatgagcatgg-3'
   b. The sequence between mouse Adam6a and Adam6b is deleted by targeting the Bsd cassette to RP23-393F3 (Fig. 4c). In such a recombineered product, ~5kb upstream sequence of and ~5kb downstream sequence of Adam6b and Adam6a respectively are still kept to maintain their specific regulation of expression in mouse cells.
2. Mouse Adam6a and Adam6b are retrieved to the 5' modifying vector of the IGH BAC by homologous recombination.
   a. 5' homology arm located at ~ 5kb downstream of Adam6a or 3' homology arm located at ~5kb upstream of Adam6b gene are created by PCR using RP23-393F3 as a template (Fig. 5a).
      5' Arm: 
5'- tttatgtactataccatctcagaaagtcaggttagtctcactagcatcgtaaaagctctgtctgggcttttc
catctgctctgctttttgtctctgtgtctaaaaatatataaaccaatgttgtccagccaaaaaaaaaaaattaa
agagcaaaaggaggtaaaatggatacaaattggaaaagaagaaatcaaaatatcactacttgaagatagt
ataatatatttaactgaccacaaaaattccaccagaaaactcctaaacctgataaacaaactcagaaaaatgg
ctagatataaactta-3'
      3' Arm: 
5'- acccatagagagaaaacaggtgagttagtgcattaaaggggctgagcagggagttctcatcgctccccagcac
cagaaataagagcctctccggagctgctgggacatggaatgcagatgattcggaccatcagccccacagagacctt
tcccactctggctcagaaagaggcactggaccacagttggagaggagaatcgaaagctgatatctctgtattcactt
agcctgttacccacccatgcacccaagtccaaggtgggagaaacactgagggtctaaacacagccccagagcaac
tgccagtattaaat-3'
   b. Two homology arms are cloned into the 5' modification vector (the vector being based on pBR322). This 5' modification vector has the gene sopC (required to ensure that each daughter cell gets a copy of the plasmid), homology arm, loxP, Neo cassette, loxP 2272, PGK promoter, PB5' LTR and the homology arm: 
5'- attcaggcagttaattgttgggttcatgttttacaactaaagaataaattcaggccagatgcagtggat
catcgctataatcacaccactttcagaagcaaaaatgagggaaatcccgtgagacgaggcaatcgaagc
caacctgagcaacataaagagatgctatttctctgaaaaaatattttaaagaataagcaggtgaggggtg
gcgttcccctctacttctagatactcaggaagcaaagatgggaagattatgtgagccaggtgttcaaaatt
acagtgagctttgatcatacaactgttcttcaaactgtgcaacagggtgagagcctgtctctaaaaacaaa
taaaaaagaatcaat-3'
 of the final Human IGH BAC (Fig 5b).
   c. BAC sequence from ~ 5kb downstream of Adam6a gene to ~5kb upstream of Adam6b gene is retrieved into the 5' modifying vector of the IGH BAC by standard recombineering (Fig.5c).
   d. After retrieving, the targeting vector is constructed by removing the Bsd gene through Ascl digestion and self-ligation (Fig. 5d).
3. The retrieved Adam6a & Adam6b along with the 5'modifying cassette (Fig. 6a) is targeted into the IGH BAC (Fig 6b) through standard recombineering to generate the final IGH BAC (Fig. 6c).

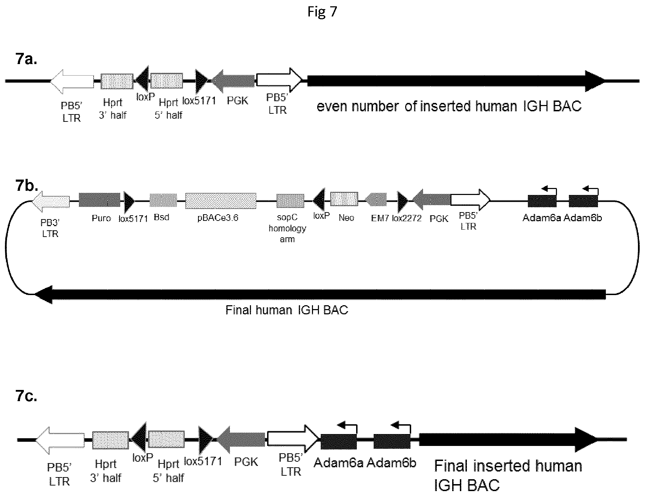

Mouse Adam6a and Adam6b along with the final human IGH BAC are inserted into mouse genome by recombinase-mediated cassette exchange (RMCE), as shown in figure 7a to 7c and as described in WO2011004192 (the disclosure of which is incorporated herein by reference). The inserted Adam6a and Adam6b can rescue the Adam6-deficient phenotype as per the present invention.

### Example 4: Fertile Mice & Progeny Comprising ADAM6 Genes

Using recombineering and ES cell genomic manipulation, mouse AB2.1 embryonic stem cell genomes were engineered to insert varying repertoires of human variable region gene segments upstream of endogenous mouse constant regions in endogenous IgH loci to functionally replace endogenous mouse variable regions. The endogenous VDJ region was deleted from the IgH loci, thereby removing the ADAM6a and ADAM6b genes from the loci. Expressible mouse ADAM6a and ADAM6b genes with wild-type promoters were inserted upstream of the IgH locus on mouse chromosome 12. Progeny mice were developed that were heterozygous for the IgH transgene (ie, having genomes with one copy of the transgenic IgH locus and with the other IgH locus rendered non-functional). Fertile heterozygous mice were obtained and bred together to produce homozygous progeny. These progeny were homozygous for the IgH transgene having the ADAM6 deletion and also homozygous for the inserted mouse ADAM6a and 6b genes. Moreover, we obtained fertile male and female homozygotes that were able to breed and produce progeny. A summary is provided below.

Three different homozygous lines were produced: IgH 1 mice; IgH 2 mice and IgH3 mice. These mice were homozygous for deletion of ADAM6 genes from the endogenous mouse IgH locus, homozygous for insertion of mouse ADAM6a and ADAM6b genes on chromosome 12 (upstream of the IgH locus) and homozygous for a heavy chain transgene as follows.
IgH 1 transgene:
   comprises human heavy gene segments V_{H}2-5, V_{H}7-4-1, V_{H}4-4, V_{H}1-3, V_{H}1-2, V_{H}6-1, D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6.
IgH 2 transgene:
   comprises human heavy gene segments V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6.
IgH 3 transgene:
   comprises human heavy gene segments VH2-26, VH1-24, VH3-23, VH3-21, VH3-20, VH1-18, VH3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6.

In order to assess whether or not mice were capable of breeding, we set up various test crosses between homozygote males and fertile female mice as follows:-

| | | NUMBER OF LITTERS | TOTAL NUMBER OF PROGENY | AVERAGE NUMBER OF PROGENY |
|---|---|---|---|---|
| Control Crosses | Wild-type (WT) male x fertile female | 21 | 153 | 7.3 ± 2.3 |
| IgH 1 Test Crosses | Homozygous IgH 1 male x fertile female | 17 | 132 | 7.8 ± 2.5 |
| IgH 2 Test Crosses | Homozygous IgH 2 male x fertile female | 5 | 35 | 7.0 ± 5.2 |
| IgH 3 Test Crosses | Homozygous IgH 3 male x fertile female | 22 | 162 | 7.4 ± 3.2 |

Thus, we were able to show the production of fertile male and female mice that were either heterozygous or homozygous for the heavy chain transgene and the deletion of endogenous VDJ. Furthermore, these mice were either heterozygous or homozygous for inserted ADAM6.

In addition, the litter size of test crosses is not significantly changed (7.7 ± 3.5 mice as an average for all test crosses) from that of matings using wild-type males (8.1 ± 3.1 mice).

In further experiments, we immunised homozygous test mice with human antigens and observed a specific immune response. Both prime-boost and RIMMS immunisation protocols were used. We isolated antigen-specific B-cells and antibodies from such mice as well as nucleic acid sequences encoding such antibodies and their chains and variable regions. Furthermore, we successfully produced hybridomas from such antigen-specific B-cells.

SEQUENCE LISTING

RAT
Rattus norvegicus
Adam6

NCBI Reference Sequence: NM_138906.1

SEQ ID NO:1

ATGTTATCTCTGACCTGGGGTATGAAGCTAGTGGAAAGATCTGTGGTCCCCAGGGTCCTCCTCTTGCTCTTTGCA
CTCTGGCTGCTCCTCCTGGTTCCAGTCCGGTGTTCTGAAGGCCACCCCACTTGGCGCTACATCTCATCAGAGGTG
GTTATTCCTCGGAAGGAGATCTACCACAGCAAAGGAATTCAAACACAAGGACGGCTCTCCTATAGCTTGCGTTTT
AGGGGCCAGAGACATATCATCCACCTGCGAAGAAAGACACTAATTTGGCCCAGACACTTGTTGCTGACAACTCAG
GATGACCAAGGAGCCTTACAGATGGATTACCCTTTTTTCCCTGTAGATTGTTACTATTTTGGCTACCTAGAGGGA
ATCCCTCAATCCATGGTCACTGTGAATACTTGCTATGGAGGCCTGGAAGGGATCATGATGTTGGATGACCTTGCC
TATGAAATCAAACCCCTCAACGATTCACAGGGGTTTGAACACATTGTTTCTCAGATAGTATCAGAGCCTGATGTA
ACAGGGCCTACAAATACATGGAAACGCTTGAACCTTAATACAGGTCCTCCCTTATCCAGGACAGAGTATGCCAAT
GGAACTCCCAGAATGTCTAGTAAGAACTACGCTTCACATCCAGCTGCTATAAAAGGCCAATTCCAAGCAACTAAT
TCTATATATAAGGAAAGCAACAATATTGATACTGCGGCCAGGTATTTGTTTGAGCTCCTTAGTATAACGGACAGC
TTTCTGATCACTATTCATATGCGGTACTATGCTATTCTCTTAACTGTGTTTACCGAGAGCGATCCATTTGCACTA
GAGTATACGGTACCAGGGGGCTCTATTTATAACTATTATGTGTCTAACTTTTTTAATCGGTTGAGGCCTGATGCA
TCAACCGTACTTAATAAAGATGGGCCCTCGGATAACGACTTTCATCCAGTTGAACAGAGTTTATGTACTCCCGCA
GGCCTGACGATTGTTGGTCAACACAGACGAAGTTTTCTAGCTCTATCTGTTATGATCACCAATCGTATTGCGATG
TCTTTAGGTATAAAAGCTGATGATGAGACTTACTGCATCTGCCACAGAAGGACCACTTGCATTATGTACAAAAAC
CCTGAAATAACAGATGCTTTCAGCAATTGCTCCCTTGTGCAGATAAACCAGATACTGAATACCCCTGGTACAATG
TCATGCCTTTTCTATGACCACCATGTTTATCATAATATAACAAAAACCTACAGGTTTTGTGGAAACTTCAAGATA
GATATCGGTGAGCAGTGTGACTGTGGCTCACATAAGGCATGTTACGCAGATCCCTGCTGCGGAAGTAATTGCAAG
TTAACTGCTGGTAGCATTTGTGATAAAGAATTATGCTGTGCAAACTGCACCTACAGTCCTTCTGGGACACTCTGC
AGACCGATCCAGAACATATGTGATCTTCCAGAATACTGTAGTGGGAATAATATCTTTTGCCCTGCAGACACTTAT
CTGCAAGATGGGACGCCATGCTCAGAAGAGGGGTACTGCTATAAAGGCAACTGCACAGATCGCAGTGTGCAGTGC
AAGGAAATCTTTGGTATGAATGCTAAGGGTGCTAATATCAAGTGCTATGACATCAACAAACAACGGTTTCGATTT
GGGCACTGCACTAGAGCACAAGAGAGCCTCATGTTTAATGCTTGCTCTGATCATGATAAACTGTGTGGAAGGCTG
CAGTGTACCAACGTCACCAATCTTCCATTCCTGCAGGAACATGTTTCATTCCATCAATCGGTTATCTCTGGGTTT
ACCTGCTTTGGGCTTGATGAACATCGTGGGACAGAAACAACGGATGCTGGGCTGGTGAAACATGGTACCCCTTGC
TCCCAAACTAACTTCTGCGATCGAGGAGCTTGCAATGGAAGTTTATCTCGGTTGGATTATGACTGCACCCCAGAA
AAATGCAATTTTAGAGGAGTGTGTAATAATCATCGGCATTGCCATTGTCATTTAGGTTGGAAACCTCCTCTGTGC
AGAGAGGAGGGGCCTAGCGGGAGCACGGACAGTGGGTCCCCTCCGAAGGAAAGGCGCACAATAAAACAGAGCAGA
GAACCACTGTTATATTTAAGAATGCTCTTTGGTCGTCTTTATTTATTCATTGTCTCGCTGCTCTTTGGAGTGGCC
ACTCGCGCAGGAGTTATTAAGGTCTTTAAGTTTGAAGACTTGCAAGCTGCTCTGCGGGCTGCACAAGCCAAGGCG
ACTTAA

RABBIT
Oryctolagus cuniculus
Adam6

NCBI Reference Sequence: NM_001165916.1

GATGCAGGAGCTATAATTAACAAGAATTCGCCAGGAGATGATGCTGTTAATCCAGCTGAGAGGAGTATATGTTCT
CCCTCAGCCCTAATTTGTCTTGGTCAACATGGTCGAAATCCTTTATTTTTATCTATTATAATAACCAATCGTGTT
GGAAGGAGTTTAGGCCTAAAACATGATGAGGGGTACTGTATCTGCCAGAGAAGGAACACCTGCATCATGTTCAAA
AATCCTCAATTAACAGATGCTTTCAGCAATTGTTCCCTTGCAGAGATAAGCAACATACTTAATACTCCTGATCTG
ATGCCATGTCTTTTCTATGACCGTCATGTTTATTATAATACATCATTGACTTATAAGTTTTGTGGAAACTTCAAA
GTAGATAACAATGAGCAGTGTGACTGTGGCTCCCAAAAGGCATGTTATTCAGATCCCTGCTGTGGAAATGATTGC
AGGTTAACACCTGGTAGCATTTGTGATAAAGAATTATGCTGTGCAAATTGCACTTACAGTCCTTCTGGGACACTC
TGCAGACCTATCCAGAACATATGTGATCTTCCAGAGTACTGTAGTGGCTCTAAGTTCATTTGCCCAGATGACACT
TATCTGCAAGATGGGACACCATGCTCAGAAGAGGGTTACTGCTATAAAGGTAACTGCACTGATCGCAACATACAA
TGCATGGAAATCTTTGGTGTAAGTGCTAAGAATGCTAATATTAAGTGCTATGACATCAACAAACAACGGTTTCGA
TTTGGGCATTGTACTAGAGCAGAAGAAAGCCTCACATTCAATGCTTGTGCTGATCAGGACAAGCTGTGTGGAAGG
TTGCAGTGTACCAATGTCACCAATCTTCCATTTTTGCAAGAACATGTTTCATTCCATCAATCGGTTATCTCTGGG
GTTACCTGCTTTGGGCTTGATGAACATCGTGGGACAGAAACAGCAGATGCTGGATTGGTGAGACATGGTACCCCG
TGTTCAAGGGGTAAGTTCTGTGATCGAGGAGCTTGCAATGGAAGTTTATCTCGTTTGGGTTATGACTGCACCCCA
GAAAAATGCAATTTCAGAGGAGTGTGTAACAATCGTCGGAATTGCCATTGCCATTTTGGTTGGAGCCCTCCAAAG
TGCAAAGAAGAGGGACACAGTGGGAGCATAGACAGTGGGTCCCCTCCGGTTCAAAGGCGCATAATAAAACAGAAC
CTAGAGCCAGTAGTGTATTTAAGAATACTCTTTGGTCGTATTTACTTCCTCTTTGTTGCACTGCTCTTTGGCATT
GCCACTCGTGTAGGAGTTACTAAGATATTTAGGTTTGAAGACTTGCAAGCTGCTTTACGTTCTTGGCAAGAACAA
GCAAAGGACAAGTAA

Mus musculus

Adam6b

NCBI Reference Sequence: NM_001009545.1

SEQ ID NO:4

ATGTTATCTCTGACCTGGGGCATGAGGCTAGTGGAAAGACCTGTGGTCCCCAGGGTCCTCCTCTTGCTAT
TTGCACTCTGGCTGCTCCTCCTGGTTCCAGTCTGGTGTTCTCAAGGCCATCCTACTTGGCGTTACATCTC
ATCGGAGGTGGTTATTCCTCGGAAGGAGATCTACCATACCAAAGGACTTCAAGCACAAAGACTGCTCTCA
TATAGCTTGCATTTTCGGGGCCAGAGACATATCATCCACCTGCGGAGAAAGACACTCATTTGGCCCAGAC
ACTTGTTGCTGACAACTCAAGATGACCAAGGAGCCTTACAGATGGATTACCCCTTTTTTCCTGTAGATTG
TTACTATATTGGCTACCTGGAGGGGATCCCACAATCCATGGTCACTGTGGATACTTGTTATGGGGGCCTG
TCAGGGGTCATGAAGTTAGATGACCTTACCTATGAAATCAAACCCCTCAATGATTCACAGAGCTTTGAAC
ACCTTGTTTCTCAGATAGTATCTGAGTCTGATGACACAGGGCCTATGAATGCATGGAAGCACTGGAGCCA
TAATACAGGTTCTCCCTCCTCCAGATTGGAATATGCAGATGGAGCTCCCAGAATATCTAGTAAGAACTAC
GCTACACATCCAGCTGCTATAAAAGGCCACTTTCAAGCAACCAATTCTGTATATAATTCTGCTGCAGGTG
ACAAACTTTCATCTACTGTTGGGTATTTGTTTCAAGTCATTAGTTTAATGGACACCTATCTGACCAATCT
TCATATGCGGTACTATGTCTTTCTCATGACTGTGTACACCAATTCTGATCCATTTCGACTTGAGTTTGCA
GTTCCAGGAGGGTCGGCTTATAATTACTATGTGTCAGTCTTTTATAATAAATTTAAGCCTGATGCAGGAG
TTTTACTTAATAAGTATGGGCCACAAGATAACCAGGTTAATCCAGCTGAGAGGAGTATATGTTCTTCCTT
AGCCCTAATTTGTATTGGTAAATATGATCGAAATCCTTTATTTTTATCTCCTATAATAACCAATCGTGTT
GGAAGGAGTTTAGGCTTAAAATATGATGAGGGGTACTGTGTCTGCCAGAGAAGGAACACCTGCATTATGT
TCAGACATCCTCAATTAACAGATGCTTTCAGCAATTGTTCCCTTGCAGAGATAAGCAACATACTTAATAC
TCCTGGTCTGATGCCATGTCTTTTCTATGACCGTCATGTTTATTATAATACATCATTGACTTATAAGTTT
TGTGGAAACTTCAAAGTAGATAACGATGAGCAGTGTGACTGTGGCTCCCAAAAGGCATGTTATTCAGATC
CCTGCTGTGGAAATGATTGCAGGTTAACACCTGGTAGCATTTGTGATAAAGAATTATGCTGTGCAAATTG
CACTTACAGTCCTTCTGGGACACTCTGCAGACCTATCCAGAACATATGTGATCTTCCAGAGTACTGTAAT
GGGACTAAATACATTTGCCCAGATGACACTTATCTGCAAGATGGGACACCATGCTCAGAAGATGGTTACT
GCTATAAAGGTAACTGCACTGATCGCAACATACAATGCATGGAAATCTTTGGTGTAAGTGCTAAGAATGC
TAATATTAAGTGCTATGACATCAACAAACAACGGTTTCGATTTGGGCATTGTACTAGAGCAGAAGAAAGC
CTCACATTCAATGCTTGTGCTGATCAGGACAAGCTGTGTGGAAGGTTGCAGTGTACCAATGTCACCAATC
TTCCATATTTGCAAGAACATGTTTCATTCCATCAATCGATTATCTCTGGGTTTACCTGCTTTGGGCTTGA
TGAACATCGTGGGACAGAAACAACAGATGCTGGAATGGTGAGACATGGTACCCCCTGCTCCAAAAGTAAG

TTCTGTGATCAAGGAGCTTGCAGTGGAAGTTTATCTCATTTGGGTTATGACTGCACCCCAGAAAAATGCA
GTTTTAGAGGAGTGTGTAACAATCATCGGAATTGCCATTGTCATTTTGGTTGGAAGCCTCCAGAGTGCAA
AGAAGAGGGACTAAGTGGGAGCATAGACAGTGGGTCCCCTCCAGTTCAAAGGCACACAATAAAACAAAAA
CAAGAGCCAGTGGTGTATTTAAGAATACTCTTTGGTCGTATTTACTTCCTCTTTGTTGCACTGCTCTTTG
GCATTGCCACTCGTGTAGGAGTTACTAAGATTTTTAGATTTGAAGACTTGCAAGCTACTTTACGTTCTGG
GCAAGGACCAGCAAGGGACAAGCCAAAGTAA

SEQ ID NO:5

5' Homology Arm:

tatgttgatggatttccatatattaaaccatccctgcatccctgggatgaagcctacttggtcatgataga

cgattgttttgatgtgttcttggattcagttagtgagaaatatattgagtatttttacatcgatattcataaggg

aaattggtctgaagttctctttctttgttgggtctttatgtggtttagttatca

SEQ ID NO:6
3' Homology Arm:

tgattccaccagaggttcttttatccttgagaagagtttttgctatcctaggttttttgttattccacatgaatttg

cagattgctctttctaattccttgaagaattgagttggaatttgatggggattgcattaaatctgtagattccttttgg

caagacagccatttttacaatgttaatcctgccaatccatgagcatgg

SEQ ID NO:7
5' Homology Arm:

tttatgtactataccatctcagaaagtcaggttagtctcactagcatcgtaaaagctctgtctgggcttttc

catctgctctgctttttgtctctgtgtctaaaaatatataaaccaatgttgtccagccaaaaaaaaaaaattaa

agagcaaaaggaggtaaaatggatacaaattggaaaagaagaaatcaaaatatcactacttgaagatagt

ataatatatttaactgaccacaaaaattccaccagaaaactcctaaacctgataaacaaactcagaaaaatgg

ctagatataaactta

SEQ ID NO:8
3' Homology Arm:

acccatagagagaaaacaggtgagttagtgcattaaaggggctgagcagggagttctcatcgctccccagcac

cagaaataagagcctctccggagctgctgggacatggaatgcagatgattcggaccatcagccccacagagacctt

tcccactctggctcagaaagaggcactggaccacagttggagaggagaatcgaaagctgatatctctgtattcactt

agcctgttacccacccatgcacccaagtccaaggtgggagaaacactgagggtctaaacacagccccagagcaac

tgccagtattaaat

SEQ ID NO:9
IgH BAC Homology Arm:

attcaggcagttaattgttgggttcatgttttacaactaaagaataaattcaggccagatgcagtggat
catcgctataatcacaccactttcagaagcaaaaatgagggaaatcccgtgagacgaggcaatcgaagc
caacctgagcaacataaagagatgctatttctctgaaaaaatattttaaagaataagcaggtgaggggtg
gcgttcccctctacttctagatactcaggaagcaaagatgggaagattatgtgagccaggtgttcaaaatt
acagtgagctttgatcatacaactgttcttcaaactgtgcaacagggtgagagcctgtctctaaaaacaaa
taaaaaagaatcaat

### STATEMENTS OF INVENTION:

51. A method of making a mouse cell or a fertile mouse that is homozygous for a transgenic antibody heavy chain locus, the mouse having a genome that (a) comprises each transgenic heavy chain locus on a respective copy of chromosome 12; and (b) is inactivated for endogenous antibody heavy chain expression; the method comprising the steps of (c) constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse or human heavy chain constant region (optionally Cmu and/or Cgamma); (d) simultaneously or separately from step (c), deleting all or part of the mouse endogenous heavy chain VDJ region of said chromosome 12 to inactivate endogenous antibody heavy chain expression, wherein the deletion includes mouse ADAM6 genes; (e) simultaneously or separately from step (c) or (d), inserting into the ES cell genome one or more ADAM6 genes; and optionally (f) developing the ES cell into a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

S2. A method of making a mouse cell or a fertile mouse that is homozygous for a transgenic antibody heavy chain locus, the mouse having a genome that (a) comprises each transgenic heavy chain locus on a respective copy of chromosome 12; and (b) is inactivated for endogenous antibody heavy chain expression; the method comprising the steps of (c) constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse or human heavy chain constant region (optionally Cmu and/or Cgamma); (d) simultaneously or separately from step (c), deleting all or part of the mouse endogenous heavy chain VDJ region of said chromosome 12 to inactivate endogenous antibody heavy chain expression, wherein the deletion includes mouse ADAM6 genes; (e) developing the ES cell into a child mouse or progeny thereof whose genome comprises a said transgenic heavy chain locus; (f) deriving a second ES cell from said mouse and inserting into the genome of said second ES cell one or more ADAM6 genes; and optionally (g) developing the second ES cell into a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

S3. A method of making a fertile mouse that is homozygous for a transgenic antibody heavy chain locus, the mouse having a genome that (a) comprises each transgenic heavy chain locus on a respective copy of chromosome 12; and (b) is inactivated for endogenous antibody heavy chain expression; the method comprising the steps of (c) constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse or human heavy chain constant region (optionally Cmu and/or Cgamma); (d) simultaneously or separately from step (c), deleting all or part of the mouse endogenous heavy chain VDJ region of said chromosome 12 to inactivate endogenous antibody heavy chain expression, wherein the deletion includes mouse ADAM6 genes; (e) developing the ES cell into a child mouse or progeny thereof whose genome comprises a said transgenic heavy chain locus; and (f) by breeding using said child mouse (or progeny) and a further mouse whose genome comprises one or more ADAM6 genes, developing a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

S4. Use of an ADAM6 gene in the manufacture of a mouse for producing a fertile mouse (eg, a fertile male mouse), wherein the mouse has a genome that comprises (a) a transgenic heavy chain locus comprising one or more human VH gene segments, one or more human D gene segments and one or more human JH gene segments inserted into DNA of a chromosome 12 so that the human gene segments are operably connected upstream of a mouse or human heavy chain constant region (optionally Cmu and/or Cgamma); and (b) a deletion of all or part of the mouse endogenous heavy chain VDJ region of said chromosome 12 to inactivate endogenous antibody heavy chain expression, wherein the deletion includes mouse ADAM6 genes; and wherein one or more ADAM6 genes are inserting into the genome.

S5. Use of an ADAM6 gene in the manufacture of a mouse cell for producing a cell that is capable of developing into such fertile mouse (eg, a male mouse); wherein the mouse cell has a genome that comprises (a) a transgenic heavy chain locus comprising one or more human VH gene segments, one or more human D gene segments and one or more human JH gene segments inserted into DNA of a chromosome 12 so that the human gene segments are operably connected upstream of a mouse or human heavy chain constant region (optionally Cmu and/or Cgamma); and (b) a deletion of all or part of the mouse endogenous heavy chain VDJ region of said chromosome 12 to inactivate endogenous antibody heavy chain expression, wherein the deletion includes mouse ADAM6 genes; and wherein one or more ADAM6 genes are inserting into the genome.

S6. The method or use of any preceding claim, wherein an ADAM6 gene is inserted on one or both chromosomes 12.

S7. The method or use of claim 6, wherein an ADAM6 gene is inserted (i) within one or both transgenic heavy chain loci or (ii) within 20 Mb of one or both transgenic heavy chain loci.

S8. The method or use of any preceding claim, wherein said human gene segments are inserted into chromosome 12 to replace all or part of the endogenous heavy chain VDJ region, so that insertion of the human gene segments and deletion of the endogenous VDJ DNA take place simultaneously; optionally wherein the entire endogenous VDJ region is replaced.

S9. The method or use of any preceding claim, wherein mouse ADAM6a and ADAM6b genes are inserted, such that the final fertile mouse can express both ADAM6a and ADAM6b proteins.

S10. The method or use of any preceding claim, wherein the genome of the final fertile mouse or progeny is homozygous for each inserted ADAM6 gene; optionally wherein the genome comprises more than two copies of mouse ADAM6a and/or ADAM6b genes.

511. The method or use of any preceding claim, with the alternative that each organism is a rat instead of a mouse and each said chromosome is a chromosome 6 instead of chromosome 12; or with the alternative that each cell is a rat cell instead of a mouse cell and each said chromosome is a chromosome 6 instead of chromosome 12.

S12. A mouse (optionally a male mouse) comprising a transgenic antibody heavy chain locus, the mouse having a genome that (i) comprises said transgenic heavy chain locus on a chromosome 12; and (ii) is inactivated for endogenous antibody heavy chain expression; wherein said chromosome 12 of the genome comprises (iii) a transgenic antibody heavy chain locus comprising one or more human VH gene segments, one or more human D gene segments and one or more human JH gene segments operably connected upstream of a mouse or human heavy chain constant region (optionally Cmu and/or Cgamma); (iv) a deletion of all or part of the mouse endogenous heavy chain VDJ region of said chromosome 12 to inactivate endogenous antibody heavy chain expression, wherein the deletion includes mouse ADAM6 genes; and wherein the genome comprises (v) an insertion of one or more expressible ADAM6 genes.

S13. The mouse of claim 12, wherein the genome comprises a transgenic antibody heavy chain locus on a respective copy of chromosome 12.

S14. A rat (optionally a male rat) comprising a transgenic antibody heavy chain locus, the rat having a genome that (i) comprises said transgenic heavy chain locus on a chromosome 6; and (ii) is inactivated for endogenous antibody heavy chain expression; wherein said chromosome 6 of the genome comprises (iii) a transgenic antibody heavy chain locus comprising one or more human VH gene segments, one or more human D gene segments and one or more human JH gene segments operably connected upstream of a rat or human heavy chain constant region (optionally Cmu and/or Cgamma); (iv) a deletion of all or part of the rat endogenous heavy chain VDJ region of said chromosome 6 to inactivate endogenous antibody heavy chain expression, wherein the deletion includes rat ADAM6; and wherein the genome comprises (v) an insertion of one or more expressible ADAM6 genes.

S15. The rat of claim 13, wherein the genome comprises a transgenic antibody heavy chain locus on a respective copy of chromosome 6.

S16. The mouse of claim 12 or 13 or rat of claim 14 or 15, wherein each inserted ADAM6 gene is on a (i) chromosome 12 wherein the animal is a mouse; or (ii) chromosome 6 wherein the animal is a rat.

S17. The mouse or rat of any one of claims 12 to 16, wherein an inserted ADAM6 gene is inserted (i) within one or both transgenic heavy chain loci or (ii) within 20 Mb of one or both transgenic heavy chain loci.

S18. The mouse or rat of any one of claims 12 to 17, wherein the human gene segments replace all or part of the endogenous VDJ region in each heavy chain locus.

S19. The mouse or rat of any one of claims 12 to 18, wherein the genome comprises inserted expressible mouse ADAM6a and ADAM6b genes.

S20. The mouse or rat of any one of claims 12 to 19, wherein the genome comprises an inserted expressible rat ADAM6 gene.

S21. The mouse or rat of any one of claims 12 to 20, wherein the genome is homozygous for each inserted ADAM6 gene; optionally wherein the genome comprises more than two copies of ADAM6 genes selected from rat ADAM6, mouse ADAM6a and mouse ADAM6b genes.

S22. The mouse or rat of any one of claims 12 to 21 wherein the genome comprises one or more transgenic light chain loci each comprising one or more human light chain V gene segments and one or more light chain J gene segments operably connected upstream of a light chain constant region (eg, an endogenous mouse or rat C kappa constant region).

S23. The method, use, mouse or rat of any preceding claim, wherein the genome comprises an exogenous ADAM6.

S24. The method, use, mouse or rat of any preceding claim, wherein the genome comprises ADAM6 that is inserted from a targeting vector (eg, a BAC vector).

S25. The method, use, mouse or rat of any preceding claim, wherein the genome comprises ADAM6 exons is inserted by targeted or random insertion into an ES cell or zygote.

S26. The method, use, mouse or rat of any preceding claim, wherein endogenous ADAM6 is deleted from the genome.

S27. A non-human vertebrate (optionally a mouse or rat) or non-human vertebrate cell (optionally a mouse or rat cell) having a genome that (i) comprises one or more transgenic antibody loci capable of expressing antibodies comprising human variable regions (optionally following antibody gene rearrangement); and (ii) is inactivated for endogenous antibody expression; wherein (iii) endogenous variable region gene segments have been translocated to a chromosomal species (eg, chromosome 15) that does not contain antibody variable region gene segments in wild-type vertebrates of said non-human type, whereby endogenous antibody expression is inactivated.

S28. A mouse or mouse cell having a genome that (i) comprises one or more transgenic antibody loci capable of expressing antibodies comprising human variable regions (optionally following antibody gene rearrangement); and (ii)is inactivated for endogenous mouse antibody expression; wherein (iii) a plurality of endogenous mouse variable region gene segments are absent from chromosomes 12 in the genome, but are present in germline configuration (with respect to each other) on one or more chromosomes other than chromosomes 12 (eg, the gene segments are on chromosome 15), whereby endogenous mouse antibody expression is inactivated.

S29. A rat or rat cell having a genome that (i) comprises one or more transgenic antibody loci capable of expressing antibodies comprising human variable regions (optionally following antibody gene rearrangement); and (ii) is inactivated for endogenous rat antibody expression; wherein (iii)a plurality of endogenous rat variable region gene segments are absent from chromosomes 6 in the genome, but are present in germline configuration (with respect to each other) on one or more chromosomes other than chromosomes 6 (eg, the gene segments are on chromosome 15), whereby endogenous rat antibody expression is inactivated.

S30. The vertebrate, mouse, rat or cell of any one of claims 27 to 29, wherein said genome comprises a transgenic antibody heavy chain locus (in heterozygous or homozygous state), the locus comprising one or more human VH gene segments, one or more human D gene segments and one or more human DH gene segments operably connected upstream of a non-human vertebrate (eg, mouse or rat) constant region or a human constant region (optionally Cmu and/or Cgamma); and said endogenous variable region gene segments are selected from endogenous (a) VH; (b) D; (c) JH; (d) VH and D; (e) D and JH; and (f) VH, D and JH.

S31. The vertebrate, mouse, rat or cell of any one of claims 27 to 30, wherein the genome comprises expressible endogenous ADAM6 gene(s).

S32. A male vertebrate, mouse, rat or cell of any one of claims 27 to 31.

S33. A method of making a non-human vertebrate cell (optionally a mouse or rat cell), the method comprising (i) inserting into a non-human ES cell genome one or more transgenic antibody loci comprising human variable region gene segments; and (ii) inactivating endogenous antibody expression by translocating endogenous variable region gene segments to a chromosomal species (eg, chromosome 15) that does not contain antibody variable region gene segments in wild-type vertebrates of said non-human type, whereby a non-human vertebrate ES cell is produced that is capable of giving rise to a progeny cell in which endogenous antibody expression is inactivated and wherein the progeny is capable of expressing antibodies comprising human variable regions; and (iii) Optionally differentiating said ES cell into said progeny cell or a non-human vertebrate (eg, mouse or rat) comprising said progeny cell.

S34. The method, use, vertebrate, mouse, rat or cell of any preceding claim, wherein the genome comprises an inserted human VDJ region that includes bases 105,400,051 to 106,368,585 from human chromosome 14.

S35. The method, use, vertebrate, mouse, rat or cell of any preceding claim, wherein the genome is homozygous at the Ig heavy chain, Igλ and Igκ loci.

S36. The method, use, vertebrate, mouse, rat or cell of any preceding claim, wherein the non-human vertebrate VDJ region of the endogenous heavy chain Ig locus and the VJ region of the endogenous lambda and kappa loci have been inactivated.

S37. The method, use, vertebrate, mouse, rat or cell of any preceding claim, wherein the genome comprises (i) a heavy chain transgene comprising substantially the full human repertoire of IgH V, D and J regions; and (ii) substantially the full human repertoire of Igκ V and J regions and/or substantially the full human repertoire of Igλ V and J regions.

S38. The method, use, vertebrate, mouse, rat or cell of any preceding claim, wherein the genome comprises (a) Heavy chain loci, each comprising one or more human heavy chain V gene segments, one or more human heavy chain D gene segments and one or more human heavy chain JH gene segments upstream of an endogenous non-human vertebrate constant region or a human constant region (eg, Cmu and/or Cgamma); (b) A kappa light chain locus (optionally in homozygous state) comprising one or more human kappa chain V gene segments, and one or more human kappa chain Jκ gene segments upstream of an endogenous non-human vertebrate (eg, endogenous mouse or rat) kappa constant region; and optionally (c) A lambda light chain locus (optionally in homozygous state) comprising one or more human lambda chain V gene segments, and one or more human lambda chain Jλ gene segments upstream of a lambda constant region; and (d) Wherein the vertebrate is capable of producing chimaeric antibodies following rearrangement of said loci and immunisation with an antigen.

S39. The method, use, vertebrate, mouse or cell of any preceding claim, wherein the vertebrate is a mouse having a mouse genetic background selected from 129, BALB/c, C57BL/6N, C57/BL/6J, JM8, AB2.1, AB2.2, 12955 or 129Sv.

S40. The method, use, vertebrate, mouse or cell of any preceding claim, wherein the vertebrate is a mouse and the genome comprises an insertion of human light chain kappa VJ targeted into mouse chromosome 6 between coordinates 70,673,899 and 70,675,515, suitably at position 70,674,734, or an equivalent position in the lambda mouse locus on chromosome 16.

S41. The method, use, vertebrate, mouse, rat or cell of any preceding claim, wherein the genome comprises a lambda antibody transgene comprising all or part of the human Igλ locus including at least one human Jλ region and at least one human Cλ region.

S42. The method, use, vertebrate, mouse, rat or cell of any preceding claim, wherein the genome comprises a lambda transgene comprising a human Eλ enhancer and/or a kappa transgene comprising a human Eκ enhancer.

S43. The method, use, vertebrate, mouse, rat or cell of any preceding claim, wherein the genome comprises human DNA (as a source of heavy and/or light chain gene segments) from a library selected from a human RPCI-11 library, RPCI-13 library, CalTech A library, CalTech B library, CalTech C library or CalTech D library.

S44. The method, use, vertebrate or cell of any preceding claim, wherein the vertebrate is a mouse, rat, rabbit, Camelid or shark.

S45. The method, use, vertebrate, mouse or rat of any preceding claim, wherein the vertebrate is able to generate a diversity of at least 1 × 10⁶ different functional chimaeric antibody sequence combinations.

546. The method, use, vertebrate, mouse, rat or cell of any preceding claim, wherein human VH which is naturally inverted or pseudogene VH is omitted from the genome.

S47. A method of isolating an antibody or nucleotide sequence encoding an antibody, the method comprising (a) immunising a vertebrate (eg, mouse or rat) as recited in preceding claim with a human target antigen such that the vertebrate produces antibodies; and (b) isolating from the vertebrate an antibody that specifically binds to said antigen and/or isolating a nucleotide sequence encoding at least the heavy and/or the light chain variable regions of such an antibody; and optionally subsequently joining the variable regions of said antibody to a human constant region.

548. The method of claim 47, wherein a prime and boost protocol is used for the immunisation.

S49. The method of claim 47, wherein a RIMMS protocol is used for the immunisation.

S50. The method of any one of claims 47 to 49, comprising after step (b) mutating the amino acid sequence of the heavy and/or the light chain variable regions of the antibody to improve affinity for binding to said antigen.

S51. The method of any one of claims 47 to 50, comprising after step (b) mutating the amino acid sequence of the heavy and/or the light chain variable regions of the antibody to improve one or more biophysical characteristics of the antibody, eg, one or more of melting temperature, solution state, stability and expression (eg, in CHO or *E coli*)*.*

S52. A 4-chain antibody, a H2 antibody or an antibody fragment (eg, Fab or Fab₂) produced by the method of any one of claims 47 to 51, wherein the antibody or fragment specifically binds a human target antigen and comprises human heavy chain variable regions and a constant region; or a derivative of said antibody or fragment.

S53. An antibody produced by affinity maturing the variable regions of an antibody isolated from a vertebrate as recited in any one of claims 1 to 46, wherein the antibody specifically binds a human target antigen.

S54. An antibody produced by mutating the constant regions of an antibody isolated from a vertebrate as recited in any one of claims 1 to 46 (eg, to ablate or enhance Fc function), wherein the antibody specifically binds a human target antigen.

S55. The antibody of claim 52, 53 or 54 (eg, in fully humanised form) for use as a therapeutic or prophylactic medicament.

556. Polyclonal antibodies produced in response to antigen challenge in vertebrates according to any one of claims 1 to 46.

S57. The antibodies of claim 56 (eg, in fully humanised form) for use as a therapeutic or prophylactic medicament.

558. An antibody isolated from a vertebrate as recited in any one of claims 1 to 46, or a derivative or fragment thereof, for use in medicine, wherein the antibody specifically binds a human target antigen.

559. Polyclonal antibodies isolated from vertebrates according to any one of claims 1 to 46, for use in medicine.

S60. A human variable region (eg, a domain antibody) generated from a heavy or light chain variable chain produced by the method of any one of claims 47 to 51 and which specifically binds a human target antigen.

S61. A nucleotide sequence encoding an antibody, fragment, derivative or variable region according to any one of claims 52 to 60, optionally wherein the nucleotide sequence is part of a vector.

S62. A pharmaceutical composition comprising - an antibody isolated from a vertebrate as recited in any one of claims 1 to 46, or comprising a derivative of said antibody; or comprising polyclonal antibodies isolated from vertebrates of any one of claims 1 to 46 and - a diluent, excipient or carrier; - optionally wherein the composition is contained in an IV container (eg, and IV bag) or a container connected to an IV syringe, wherein the antibody(ies) specifically bind(s) a human target antigen.

S63. A cell line (eg, an immortalised cell line, ES cell or iPS cell line), B-cell, hybridoma or stem cell derived from a vertebrate as recited in any one of claims 1 to 46.

S64. The stem cell of claim 63 wherein the cell is a BALB/c, JM8, AB2.1 or AB2.2 embryonic stem cell.

565. The cell line, cell or hybridoma of claim 63 or 64, wherein the vertebrate is a mouse BALB/c, C57BL/6N, C57BL/6J, 129S5 or 129Sv strain.

## Claims

1. A method of making a fertile mouse that is homozygous for a transgenic antibody heavy chain locus, the mouse having a genome that
(a) comprises each transgenic heavy chain locus on a respective copy of chromosome 12; and
(b) is inactivated for endogenous antibody heavy chain expression;
the method comprising the steps of
(c) constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse endogenous heavy chain constant region (optionally C mu and/or C gamma), wherein the insertion comprises, in germline configuration, all of the V, D and J regions and intervening sequences from a human;
(d) simultaneously or separately from step (c), deleting all or part of the mouse endogenous heavy chain VDJ region of said chromosome 12 to inactivate endogenous antibody heavy chain expression, wherein the deletion includes mouse ADAM6-encoding nucleotide sequences;
(e) simultaneously or separately from step (c) or (d), inserting into the ES cell genome one or more ADAM6-encoding nucleotide sequences; and
(f) developing the ES cell into a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

2. A method of making a fertile mouse that is homozygous for a transgenic antibody heavy chain locus, the mouse having a genome that
(a) comprises each transgenic heavy chain locus on a respective copy of chromosome 12; and
(b) is inactivated for endogenous antibody heavy chain expression;
the method comprising the steps of
(c) constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse endogenous heavy chain constant region (optionally C mu and/or C gamma), wherein the insertion comprises, in germline configuration, all of the V, D and J regions and intervening sequences from a human;
(d) simultaneously or separately from step (c), deleting all or part of the mouse endogenous heavy chain VDJ region of said chromosome 12 to inactivate endogenous antibody heavy chain expression, wherein the deletion includes mouse ADAM6-encoding nucleotide sequences;
(e) developing the ES cell into a child mouse or progeny thereof whose genome comprises a said transgenic heavy chain locus;
(f) deriving a second ES cell from said mouse and inserting into the genome of said second ES cell one or more ADAM6-encoding nucleotide sequences; and
(g) developing the second ES cell into a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

3. A method of making a fertile mouse that is homozygous for a transgenic antibody heavy chain locus, the mouse having a genome that
(a) comprises each transgenic heavy chain locus on a respective copy of chromosome 12; and
(b) is inactivated for endogenous antibody heavy chain expression;
the method comprising the steps of
(c) constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse or human endogenous heavy chain constant region (optionally C mu and/or C gamma), wherein the insertion comprises, in germline configuration, all of the V, D and J regions and intervening sequences from a human;
(d) simultaneously or separately from step (c), deleting all or part of the mouse endogenous heavy chain VDJ region of said chromosome 12 to inactivate endogenous antibody heavy chain expression, wherein the deletion includes mouse ADAM6-encoding nucleotide sequences;
(e) developing the ES cell into a child mouse or progeny thereof whose genome comprises a said transgenic heavy chain locus; and
(f) by breeding using said child mouse (or progeny) and a further mouse whose genome comprises one or more ADAM6-encoding nucleotide sequences, developing a fertile mouse or a progeny thereof whose genome is homozygous for said transgenic heavy chain locus and encodes ADAM6, wherein all or part of the endogenous heavy chain VDJ region has been deleted from both chromosomes 12 in the genome; optionally wherein said fertile mouse or progeny is male.

4. The method of any preceding claim, wherein an ADAM6-encoding nucleotide sequence is inserted on one or both chromosomes 12.

5. The method of any preceding claim, with the alternative that each organism is a rat instead of a mouse and each said chromosome is a chromosome 6 instead of chromosome 12.

6. The method of any preceding claim, wherein mouse ADAM6a and ADAM6b-encoding nucleotide sequences are inserted, such that the final fertile mouse can express both ADAM6a and ADAM6b proteins.

7. A mouse (optionally a male mouse) that is homozygous for a transgenic antibody heavy chain locus, the mouse having a genome that
(i) comprises each transgenic heavy chain locus on a respective copy of chromosome 12; and (ii) is inactivated for endogenous antibody heavy chain expression;
wherein each chromosome 12 of the genome comprises
(iii) a transgenic antibody heavy chain locus comprising one or more human VH gene segments, one or more human D gene segments and one or more human JH gene segments operably connected upstream of a heavy chain constant region (optionally C mu and/or C gamma), wherein the insertion comprises, in germline configuration, all of the V, D and J regions and intervening sequences from a human;
(iv) a deletion of all or part of the mouse endogenous heavy chain VDJ region of said chromosome 12 to inactivate endogenous antibody heavy chain expression, wherein the deletion includes mouse ADAM6-encoding nucleotide sequences; and wherein the genome comprises
(v) an insertion of one or more expressible ADAM6-encoding nucleotide sequences.

8. A rat (optionally a male rat) that is homozygous for a transgenic antibody heavy chain locus, the rat having a genome that
(i) comprises each transgenic heavy chain locus on a respectively copy of chromosome 6; and
(ii) is inactivated for endogenous antibody heavy chain expression;
wherein each chromosome 6 of the genome comprises
(iii) a transgenic antibody heavy chain locus comprising one or more human VH gene segments, one or more human D gene segments and one or more human JH gene segments operably connected upstream of a heavy chain constant region (optionally C mu and/or C gamma) wherein the insertion comprises, in germline configuration, all of the V, D and J regions and intervening sequences from a human;
(iv) a deletion of all or part of the rat endogenous heavy chain VDJ region of said chromosome 6 to inactivate endogenous antibody heavy chain expression, wherein the deletion includes rat ADAM6; and wherein the genome comprises
(v) an insertion of one or more expressible ADAM6-encoding nucleotide sequences.

9. The mouse or rat of any one of claims 7 or 8, or method according to any one of claims 1-6, wherein an inserted ADAM6-encoding nucleotide sequence is inserted (i) within one or both transgenic heavy chain loci or (ii) within 20 Mb of one or both transgenic heavy chain loci.

10. The method according to any one of claims 1-6, wherein mouse ADAM6a and ADAM6b-encoding nucleotide sequences are inserted, such that the final fertile mouse can express both ADAM6a and ADAM6b, or the mouse or rat of any one of claims 7-9, wherein the genome comprises inserted expressible mouse ADAM6a and ADAM6b-encoding nucleotide sequences.

11. The mouse or rat of any one of claims 7-10, or method according to any one of claims 1-6, 9 or 10, wherein the genome is homozygous for each inserted ADAM6 gene.

12. The mouse or rat of any one of claims 7-11, or method according to any one of claims 1-6, or 9-11, wherein the genome comprises one or more transgenic light chain loci each comprising one or more human light chain V gene segments and one or more light chain J gene segments operably connected upstream of a light chain constant region (eg, an endogenous mouse C kappa constant region).

13. The mouse or rat or method of claim 12, wherein the one or more transgenic light chain loci each comprise only one human light chain V gene segment and only one light chain J gene segment operably connected upstream of a light chain constant region (eg, an endogenous mouse C kappa constant region), optionally wherein there is only one such transgenic light chain locus in the mouse or rat genome.

14. A method of isolating an antibody or nucleotide sequence encoding said antibody, the method comprising
(a) immunising a mouse or rat) according to any one of claim 7-11 with a human target antigen such that the vertebrate produces antibodies; and
(b) isolating from the mouse or rat an antibody that specifically binds to said antigen and/or isolating a nucleotide sequence encoding at least the heavy and/or the light chain variable regions of said antibody; and
optionally wherein the variable regions of said antibody are subsequently joined to a human constant region.

15. A cell line (eg, an immortalised cell line, ES cell or iPS cell line), B-cell, hybridoma or stem cell derived from a mouse as recited in any one of claims 7 and 9-13.
